# EUROPEAN PATENT SPECIFICATION

(11) **EP 2 709 557 B1**
(45) Date of publication and mention of the grant of the patent: **20.06.2018**
(21) Application number: 12721676.0
(22) Date of filing: 14.05.2012
(51) Int. Cl.: A61F 2/08

(54) **APPARATUS FOR FIXATION OF AN ACL GRAFT**
VORRICHTUNG ZUR BEFESTIGUNG EINES ACL-TRANSPLANTATS
APPAREIL DE FIXATION D'UN GREFFON DE LCA (LIGAMENT CROISÉ ANTÉRIEUR)

(30) Priority: 17.05.2011 US 201113109667; 17.05.2011 US 201113109672
(43) Date of publication of application: 26.03.2014
(73) Proprietor: Biomet Sports Medicine, LLC, Warsaw, Indiana 46582 (US)
(72) Inventor: CONNER, Nathanael K., Huntertown, Indiana 46748 (US); BERELSMAN, Brian K., Warsaw, Indiana 46580 (US); DENHAM, Gregory J., Warsaw, Indiana 46582 (US); STONE, Kevin T., Winona Lake, Indiana 46590 (US); SLATER, Nicholas R., Chandler, Arizona 85248 (US); ARNETT, Jeffrey D., Gilbert, Arizona 85297 (US); BUTTERS, Joshua A., Chandler, Arizona 85224 (US)
(74) Representative: Mays, Julie
(86) International application number: PCT/US2012/037703
(87) International publication number: WO 2012/158583

(56) References cited:
- EP-A2- 2 238 944
- WO-A2-2008/002550
- DE-U1- 20 207 781
- US-A1- 2009 054 928
- US-A1- 2011 009 885
- US-B1- 6 547 778

## Description

### CROSS REFERENCE TO RELATED APPLICATIONS

This application claims priority to U.S. Utility Application Nos. 13/109,667, filed on May 17, 2011, and 13/109,672, filed on May 17, 2011.

### FIELD

The present disclosure relates to an apparatus for fixation of an anterior cruciate ligament (ACL) as defined by the claims.

### BACKGROUND

This section provides background information related to the present disclosure that is not necessarily prior art.

Ligaments are strong fibrous connective soft tissue that connect the articular ends of bones to bind them together and to facilitate or limit motion. Injuries to ligaments are common, and patients who are physically active are generally more susceptible to such ligament injuries.

The anterior cruciate ligament (ACL) of the knee joint is a ligament frequently injured by such patients. Such injuries cause instability in the knee joint which, when left untreated, may lead to degenerative arthritis. Because of this condition, ACL reconstruction may be required. Generally during ACL reconstruction, a substitute soft tissue ligament or graft is attached to the femur and/or tibia to facilitate regrowth and permanent attachment. The substitute graft can include one or more graft bundles or strands that are tensioned prior to the femoral and/or tibial fixation. WO2008/002550 describes a graft ligament strand tensioner and EP2238944 discloses an adjustable button/loop construct for tissue reconstruction.

### SUMMARY

This section provides a general summary of the disclosure, and is not a comprehensive disclosure of its full scope or all of its features.

In one form, a tensioner system for use in tensioning graft strands in a knee reconstruction procedure is provided in accordance with various aspects of the present teachings. The tensioner system includes a handle assembly, a tensioner assembly coupled to the handle assembly and a drive shaft slidably received through and supported by the handle assembly. The handle assembly includes a hollow outer handle body and an inner handle body slidably received within the outer handle body. The tensioner assembly includes an arm support member rotatably coupled to the handle assembly and first and second arm members each rotatably coupled to lateral sides of the arm support member. Each arm member includes at least one graft attachment area adapted to be coupled to a graft strand. The arm support member is configured to rotate about an axis perpendicular to a longitudinal axis of the tensioner system and perpendicular to an axis of rotation of each of the first and second arm members. The drive shaft is received through and supported by the handle assembly such that proximal and distal ends of the drive shaft extend beyond respective proximal and distal ends of the handle assembly The tensioner system comprises a ratcheting member received in the outer handle body and including a plurality of ratchet teeth in selective engagement with a pawl carried by the outer handle body. The ratchet teeth are configured to maintain a position of the outer handle body relative to the ratcheting member upon movement of the outer handle body relative to the ratcheting member.

A method for use in graft fixation in a knee reconstruction procedure is described. The method can include forming a femoral tunnel in a femur and a tibial tunnel in a tibia, where the femoral tunnel has an opening adjacent a joint space between the femur and the tibia and the tibial tunnel has an entrance at the joint space and an opposite exit spaced apart from the joint space. The graft can be positioned into the femoral tunnel and retained relative to the femoral tunnel and passed though the tibial tunnel such that the graft extends beyond the exit of the tibial tunnel. The graft can be coupled to a tensioner device. A bone engaging member of the tensioner device can be positioned relative to the tibial tunnel. The graft can be tensioned by moving a portion of the tensioner device away from the bone engaging member thereby increasing a length of the tensioner device and applying tension to the graft strands. The method can also include ungrasping the tensioner device while the tensioner device maintains the tension in the graft.

A method for use in graft strand fixation in a knee reconstruction procedure is described. The method can include forming a femoral tunnel in a femur and a tibial tunnel in a tibia, where the femoral tunnel has an opening adjacent to a joint space between the femur and the tibia and the tibial tunnel has an entrance at the joint space and an opposite exit spaced apart from the joint space. A femoral fixation member coupled to a self-locking adjustable flexible member construct can be positioned into the femoral tunnel and the graft strands can be positioned around adjustable loops of the flexible member construct extending from the femoral tunnel. Free ends of the flexible member construct can be tensioned to reduce a size of the adjustable lops and draw the graft strands into the femoral tunnel, and the graft strands can be passed though the tibial tunnel such that the graft strands extend beyond the exit of the tibial tunnel. The graft strands can be coupled to first and second tensioning arms of a tensioner device, and a bone engaging foot of the tensioner device can be aligned relative to the tibial tunnel. The graft strands can be tensioned by moving an outer handle body of the tensioner device away from the tensioning arms, which can be allowed to rotate relative to the tensioner device and each other to provide substantially equal tension in each graft strand. The method can also include maintaining the tension in the graft strands with the tensioner device and engagement of the tensioner device to the tibia independent of any input from a user of the tensioner device. A drive shaft of the tensioner device can be translated relative to the tensioning arms and outer handle body to drive an implant into the tibial tunnel to fix the tensioned graft strands relative to the tibial tunnel.

A method for use in graft strand fixation in a knee reconstruction procedure is described. The method can include forming a femoral tunnel in a femur and a tibial tunnel in a tibia, where the femoral tunnel has an opening adjacent a joint space between the femur and the tibia and the tibial tunnel has an entrance at the joint space and an opposite exit spaced apart from the joint space. The graft can be positioned into the femoral tunnel and retained relative to the femoral tunnel. The graft can be passed though the tibial tunnel such that the graft extends beyond the exit of the tibial tunnel, and the graft can be coupled to a tensioner device. A bone engaging member of the tensioner device can be positioned relative to the tibial tunnel. The tensioner device can be grasped and the graft can be tensioned by moving a handle body of the tensioner device away from the bone engaging member thereby increasing a length of the tensioner device and applying tension to the graft. The tensioner device can be used to maintain tension in the graft and alignment of a longitudinal axis of the tensioner device with a longitudinal axis of the tibial tunnel in an absence of an external force being applied to the tensioner device.

Further areas of applicability will become apparent from the description provided herein. The description and specific examples in this summary are intended for purposes of illustration only and are not intended to limit the scope of the present disclosure.

### DRAWINGS

The present teachings will become more fully understood from the detailed description, the appended claims and the following drawings. The drawings are for illustrative purposes only of selected embodiments and not all possible limitations, and are not intended to limit the scope of the present disclosure.
Figure 1 is a perspective view of an exemplary graft strand tensioner having arms in a non-deployed position in accordance with the teachings of the present disclosure;
Figure 2 is an exploded view of components of the tensioner of Figure 1 in accordance with the teachings of the present disclosure;
Figure 3 is a side view of the tensioner of Figure 1 in accordance with the teachings of the present disclosure;
Figure 4 is a sectional view of the tensioner of Figure 1 in accordance with the teachings of the present disclosure;
Figure 5 is a perspective view of an outer handle body of the tensioner having a tension release member in accordance with the teachings of the present disclosure;
Figure 6 is a perspective view of an inner handle body of the tensioner in accordance with the teachings of the present disclosure;
Figure 7 is a perspective view of an arm support member of the tensioner in accordance with the teachings of the present disclosure;
Figure 8 is a perspective view of a tensioning arm of the tensioner in accordance with the teachings of the present disclosure;
Figure 9 is a perspective view of the tensioner showing the ratcheting member in an advanced position and the tensioning member and arms in exemplary deployed or use positions in accordance with the teachings of the present disclosure;
Figure 10 is a perspective view of a ratcheting member of the tensioner in accordance with the teachings of the present disclosure;
Figure 11 is a side view of a driver shaft of the tensioner in accordance with the teachings of the present disclosure;
Figure 12 is a perspective view of an exemplary implant for use with the tensioner in accordance with the teachings of the present disclosure;
Figure 13 is a side view of the implant of Figure 12 in accordance with the teachings of the present disclosure;
Figure 14 is a sectional view of the implant of Figure 13 in accordance with the teachings of the present disclosure;
Figures 15-17 are perspective views of exemplary alternative implants for use with the tensioner in accordance with the teachings of the present disclosure;
Figures 18 and 19 depict exemplary adjustable knotless suture constructs that can be used in an ACL reconstruction procedure;
Figures 20-24 depict coupling a graft to a femur and tibia in an exemplary ACL reconstruction procedure;
Figure 25 depicts a partial section view of an exemplary implant securing graft strands to the tibia;
Figure 26 depicts a partial perspective view of an implant coupling four grafts strands to the tibia;
Figure 27 depicts a sectional view of Figure 26;
Figure 28 is a perspective view illustrating an exemplary placement of four graft strands about the implant with the tibial tunnel removed;
Figures 29-30 are views depicting another exemplary ACL reconstruction procedure;
Figures 31A-32 are views depicting another exemplary ACL reconstruction procedure;
Figures 33A-34 are views depicting an implant for coupling a graft to the tibia and an associated exemplary ACL reconstruction procedure;
Figures 35A-36 are views depicting an implant for coupling a graft to the tibia and an associated exemplary ACL reconstruction procedure;
Figures 37A-40 are views depicting exemplary implants for coupling a graft to bone in an ACL reconstruction procedure; and
Figure 41 depicts another exemplary ACL reconstruction procedure.

### DETAILED DESCRIPTION

The following description is merely exemplary in nature and is not intended to limit the present disclosure, its application, or uses. It should be understood that throughout the drawings, corresponding reference numerals indicate like or corresponding parts and features. Throughout the description, exemplary embodiments are provided so that this disclosure will be thorough, and will fully convey the scope to those who are skilled in the art. Numerous specific details are set forth such as examples of specific components, devices, systems and/or methods, to provide a thorough understanding of exemplary embodiments of the present disclosure. It will be apparent to those skilled in the art that specific details need not be employed, that exemplary embodiments may be embodied in many different forms and that neither should be construed to limit the scope of the disclosure. In some exemplary embodiments, well-known processes, well-known device structures, and well-known technologies are not described in detail.

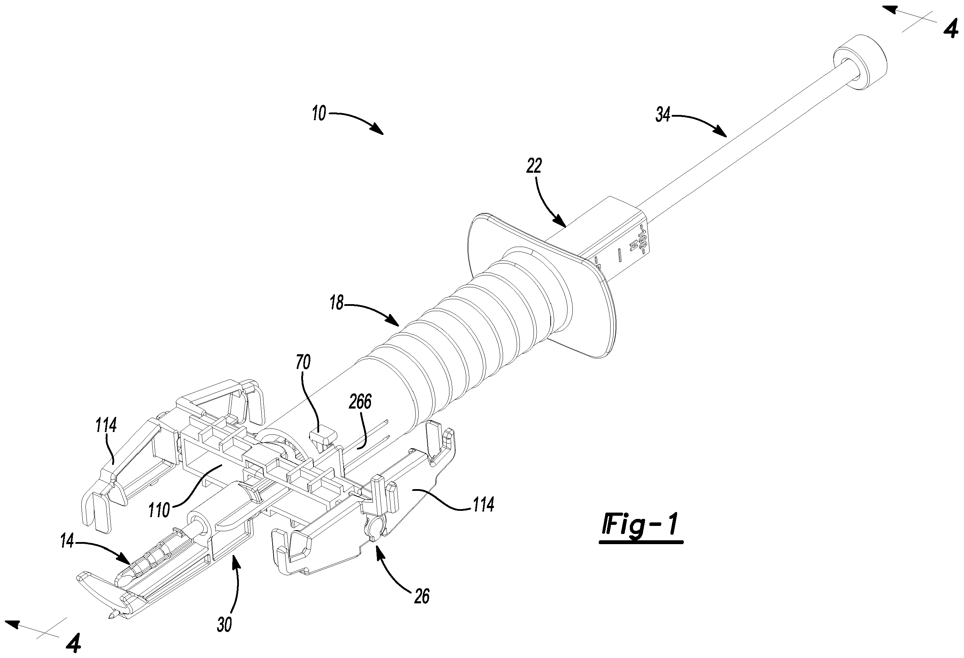

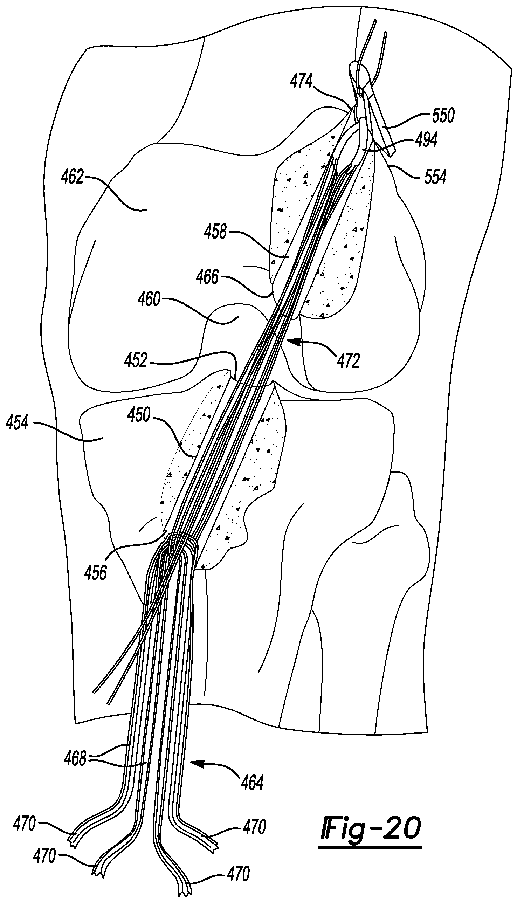

Turning now to Figures 1-14 of the drawings, an exemplary tensioner 10 and an associated implant 14 are shown in accordance with the teachings of the present disclosure. As will be discussed in greater detail below, tensioner 10 and implant 14 can be used in connection with an ACL reconstruction procedure (e.g., Figures 20-28) where one or more looped graft bundles can be tensioned and coupled relative to a tibial tunnel. As will also be discussed below, in one exemplary configuration, a surgeon can operate tensioner 10 with one hand and tensioner 10 can provide equal or substantially equal tension to four graft strands extending from the tibial tunnel.

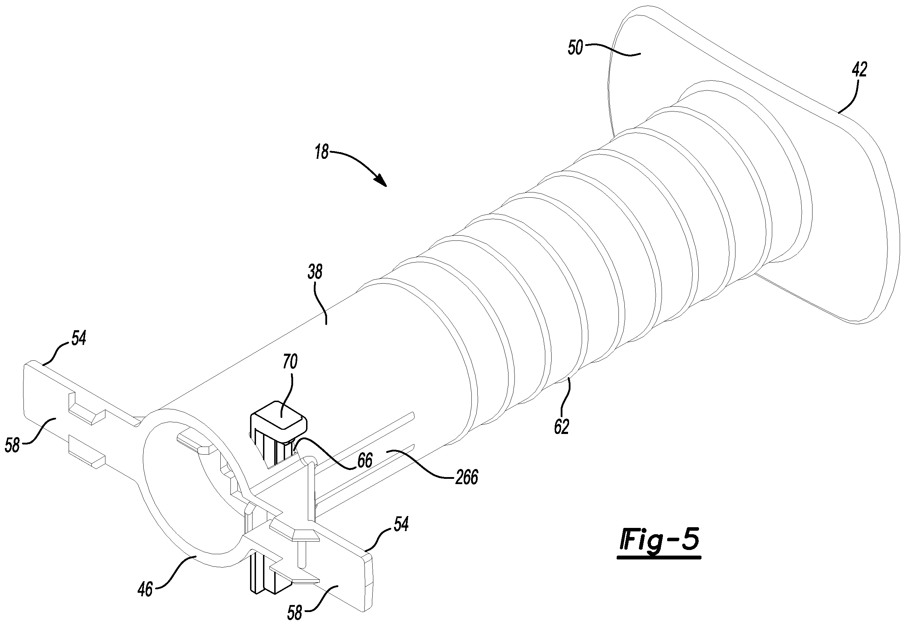

Tensioner 10 can be wholly disposable or reusable or partially disposable or reusable and can include an outer handle body 18, an inner handle body 22, a tensioning arm assembly 26, a ratcheting bone engaging member 30 and a driver shaft 34. Outer handle body 18 can include a hollow body 38 having a proximal end 42 and a distal end 46, as shown for example in Figure 5. Proximal end 42 can include a flange 50 for use in supporting a surgeon or other user's hand and distal end 46 can include a pair of outwardly extending flanges 54 having arm engaging surfaces 58. Hollow body 38 can include a plurality of ribs or other protrusions 62 to aid the surgeon in gripping outer handle body 18. An aperture 66 can be formed in body 38 for receiving a tension release member 70, which will be discussed below in greater detail.

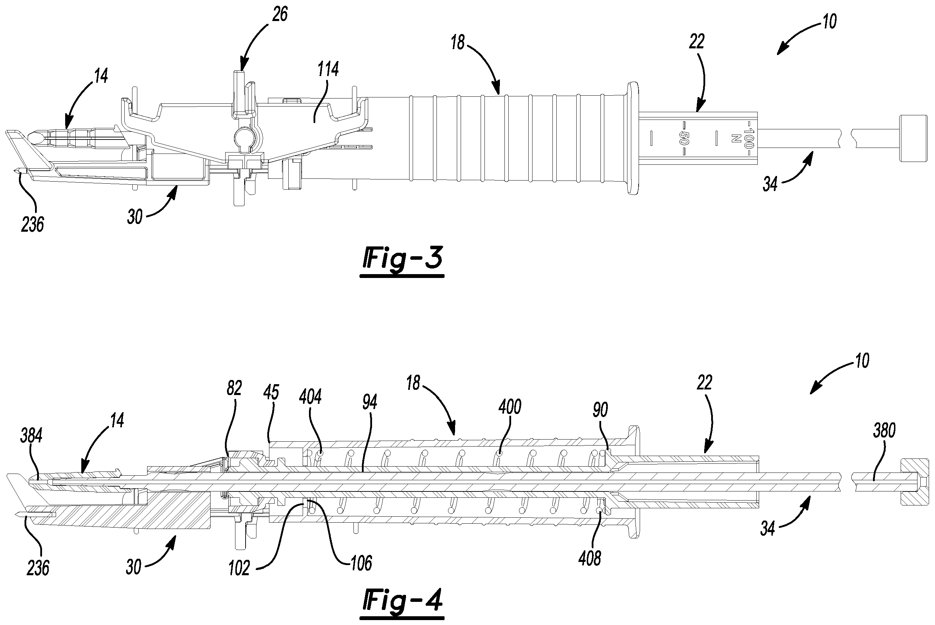

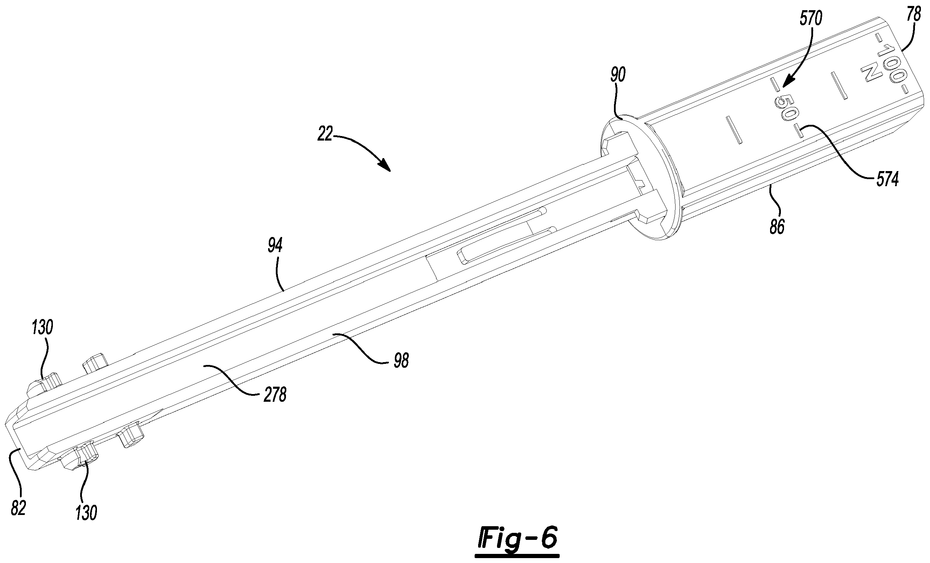

Inner handle body 22 can be received in outer handle body 18 and can include a proximal end 78 and a distal end 82, as shown for example in Figure 6. Proximal end 78 can include a first portion 86 having a flange 90 formed on a distal end thereof and a second portion 94 having a C-shaped configuration 98 in cross section extending from flange 90 to distal end 82. As will be discussed in greater detail below, the C-shaped configuration 98 of second portion 94 can axially receive driver shaft 34 therein, as shown for example in Figure 4. Outer handle body 18 can include an internal flange 102 having an aperture 106 sized and shaped to receive and support the inner handle body 22 and ratcheting member 30, as also shown in Figure 4.

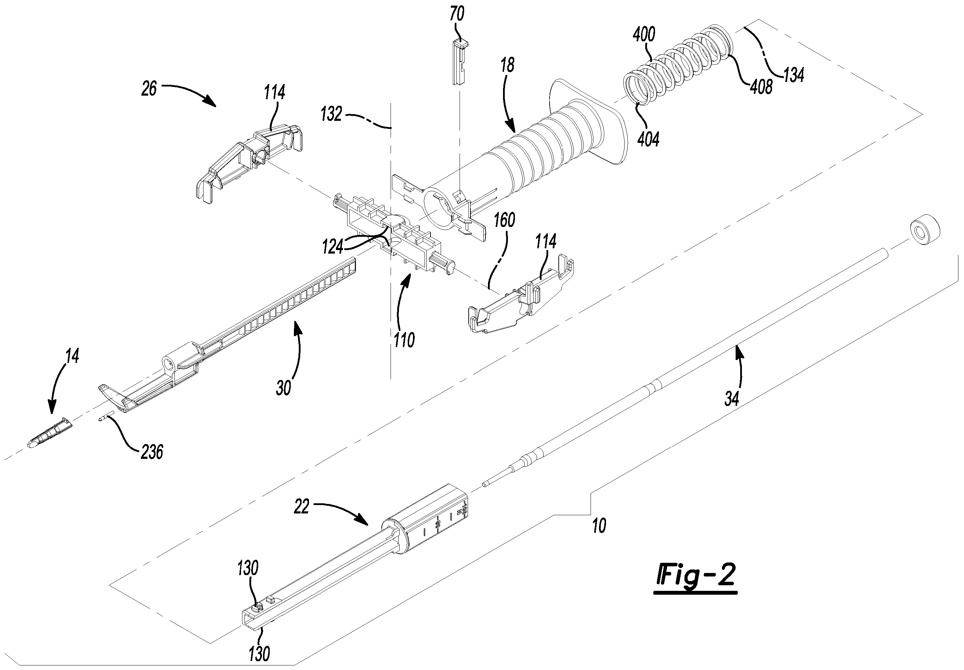

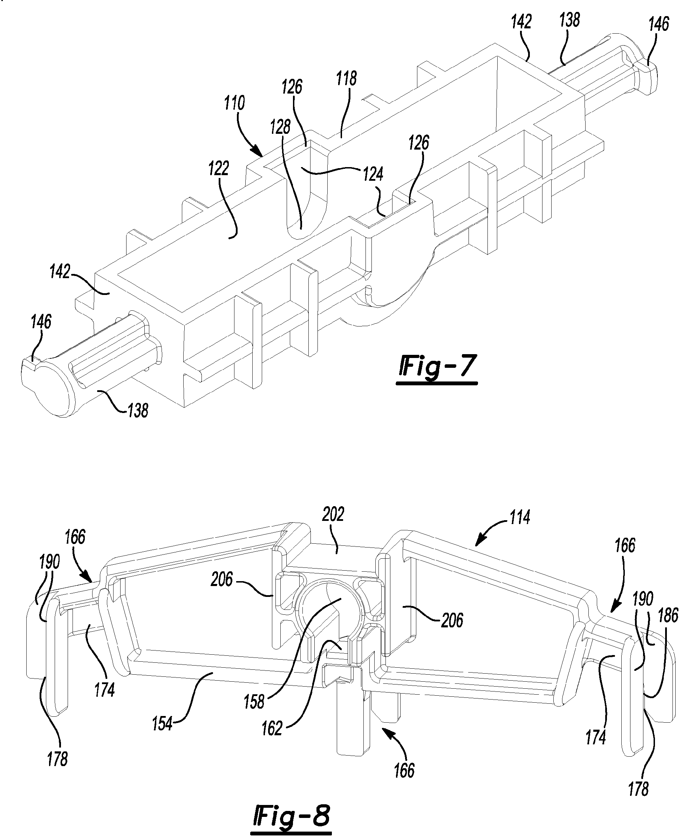

Tensioning arm assembly 26 can include an arm support member 110 and a pair of tensioning arms 114, as shown for example in Figures 7 and 8 with reference to Figure 2. Arm support member 110 can include a body 118 having a passage 122 therethrough and a pair of centrally positioned recesses 124 configured to receive a corresponding pair of projections 130 (Figure 6) extending from distal end 82 of inner handle body 22. Each recess 124 can include an open end 126 and an arcuate shaped closed end 128. As will be discussed in greater detail below, arm support member 110 can rotate or pivot about an axis 132 perpendicular to a longitudinal axis 134 (Figure 2) of tensioner 10 to cooperate with tensioning arms 114 to provide equal or substantially equal tension to graft strands that are coupled to tensioning arms 114. In the exemplary configuration illustrated, body 118 can have a rectangular shape with arm attachment members 138 extending from opposite lateral ends 142 thereof. Each arm attachment member 138 can include a flange or other retaining member 146 extending therefrom to retain the respective tensioning arm 114 thereon.

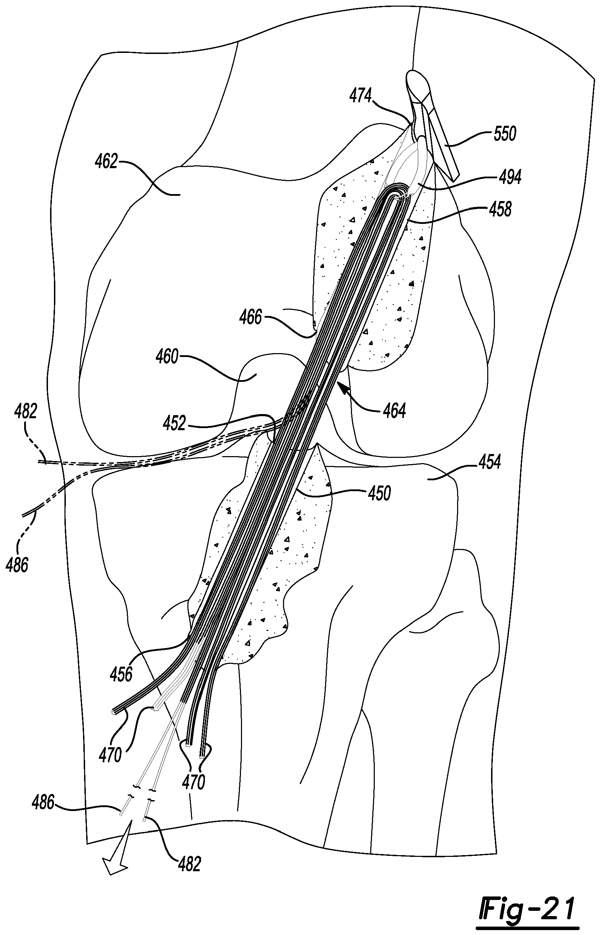

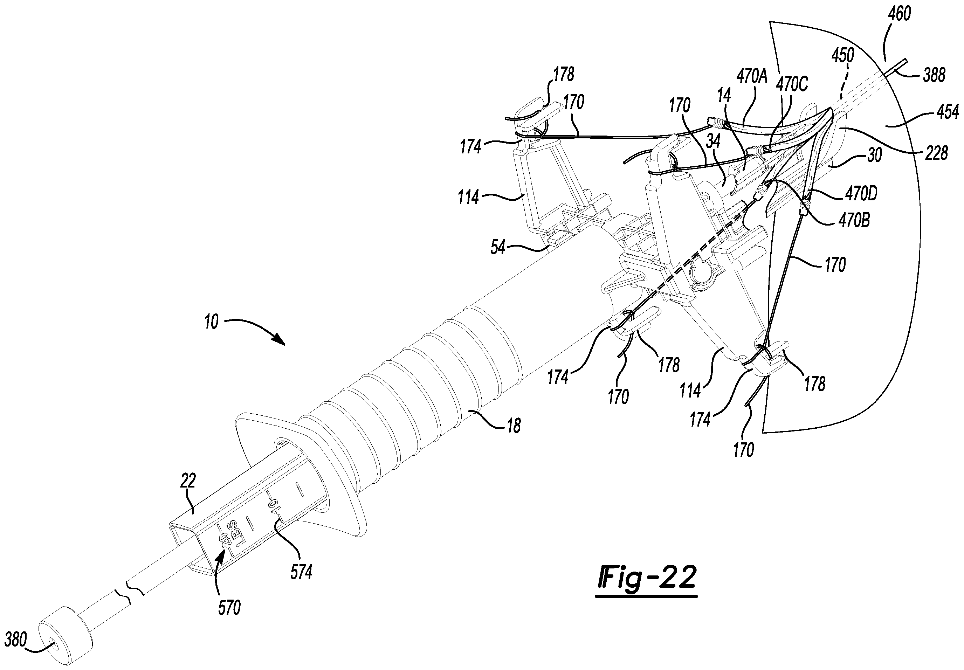

Each tensioning arm 114 can include a body portion 154 having a central aperture 158 configured to receive attachment member 138 for removably coupling tensioning arm 114 to arm support member 110. Each tensioning arm 114 can be rotatably coupled to a respective attachment member 138, where rotation of tensioning arms 114 about axis 160 aids in providing the equal or substantially equal tension to grafts strands coupled thereto. In other words, having arm support member 110 rotate about first axis 132 and tensioning arms 114 each rotate about second axis 160 allows each strand 470 (Figures 21 and 22) of the graft bundle to be separately tensioned and balanced. In the exemplary configuration illustrated, aperture 158 can include a recess 162 sized and shaped to receive retaining member 146 therethrough. Body portion 154 can include flexible member or suture attachment arrangements 166 at opposed longitudinal ends thereof to facilitate coupling the flexible member or suture 170 (e.g., Figure 22) extending from a respective graft strand thereto, as will be discussed in greater detail below.

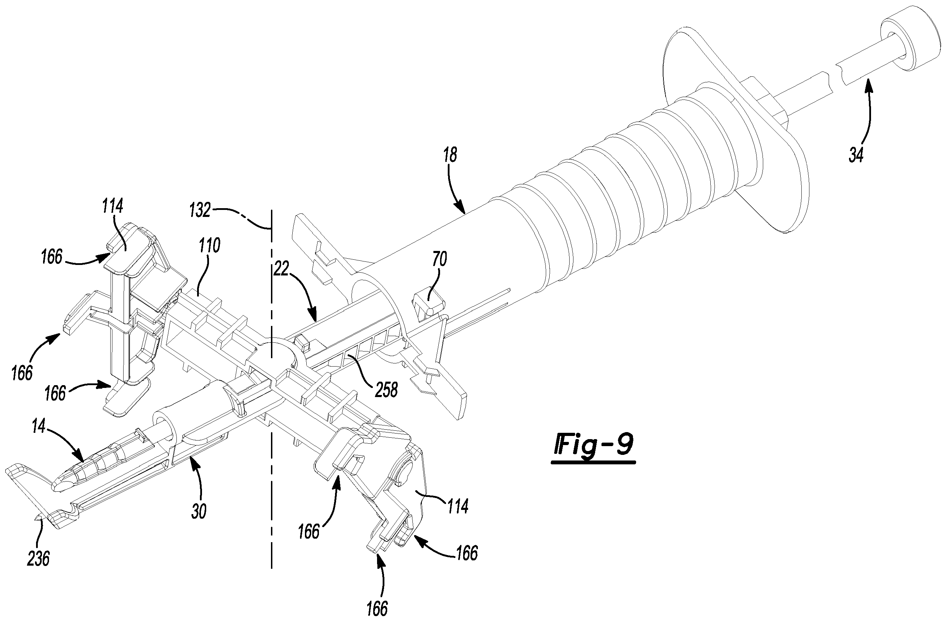

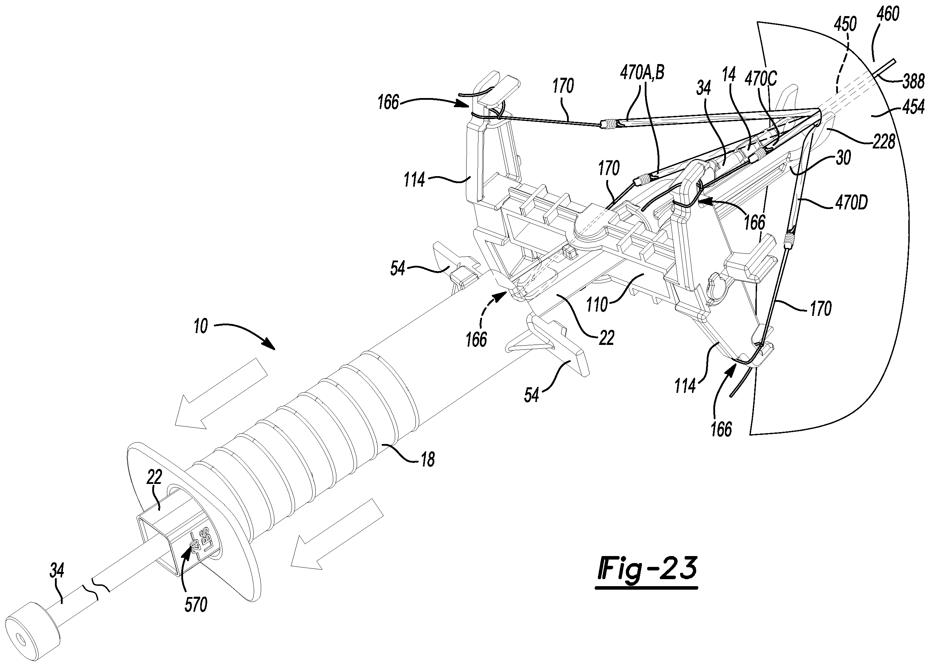

Briefly, however, each attachment arrangement 166 can include first and second attachment areas 174, 178 for use in securing the suture thereto. In one exemplary configuration, first attachment area 174 can be used to initially receive the suture 170, such as by wrapping the suture around the attachment area 174. The second attachment area 178 can include a slot 186 between adjacent flanges 190 sized and shaped to provide an interference fit to a suture received therein. In this regard, second attachment area 178 can be used to secure an end of suture 170 therein, as shown for example in Figure 23. It should be appreciated however, that first and second attachment areas 174, 178 can be used individually or in combination to removably couple a respective suture 170 thereto. In another exemplary configuration, each arm 14 can include a third attachment arrangement 166 centrally positioned between the longitudinal ends, as shown for example in Figures 8 and 9. The third attachment arrangement 166 could be used, for example, with a graft bundle having an additional two ends.

The tensioning arms 114 can also include first and second engagement surfaces 202, 206 configured to engage respective surfaces 58 of flanges 54 in the non-deployed and deployed positions of tensioning arms 114, as shown in Figure 8 with reference to Figures 1 and 22. For example, one of second engagement surfaces 206 can engage respective surfaces 58 of flanges 54 when tensioning arms 114 are in the pre-use or non-deployed position, such as a shipping or storage position, as shown for example in Figure 1. The tensioning arms 114 can be rotated to the initial use or deployed position where first engagement surfaces 202 of tensioning arms 114 engage respective surfaces 58 of flanges 54, as shown for example in Figure 22.

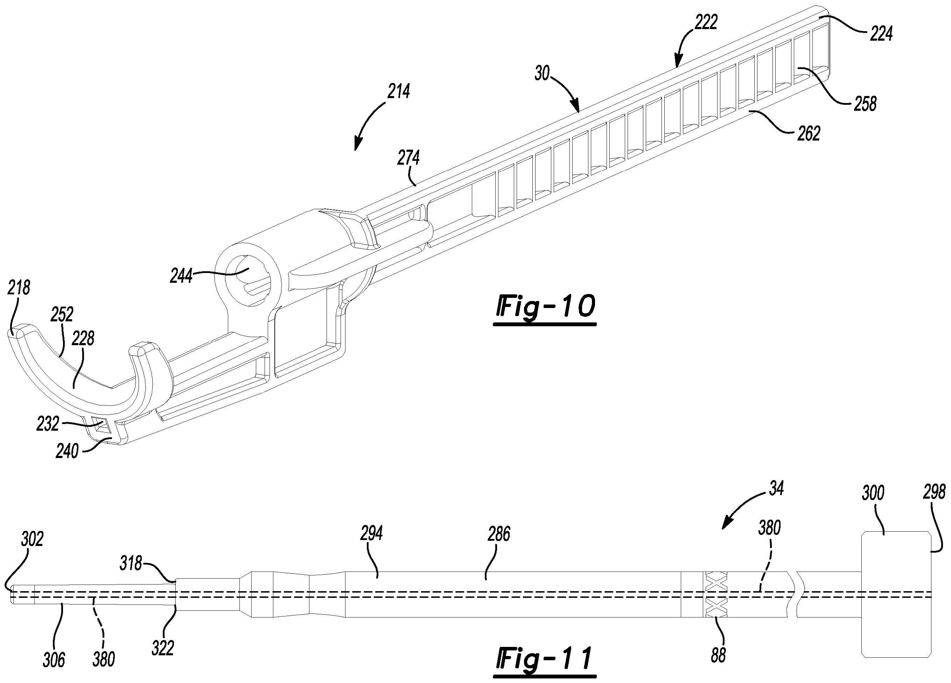

The ratcheting member 30 can include a first portion 214 defining a distal end 218 and a second portion 222 extending therefrom and defining a proximal end 224, as shown for example in Figure 10. First portion 214 can include a bone engaging member or foot 228 at the distal end 218 thereof and can include an aperture 232 for receiving a bone spike or other bone engaging or fixation member 236 (Figure 2). Bone spike 236 can include a longitudinal length so as to extend beyond an outer surface 240 of distal end 218, as shown for example in Figure 3. First portion 214 can also include a longitudinal aperture or throughbore 244 sized and shaped to receive driver shaft 34 therethrough, as will be discussed in greater detail below. In the exemplary configuration illustrated, bone engaging foot 228 can include a substantially U-shaped configuration 252 configured to align with a tibial tunnel and receive implant 14 and a portion of driver shaft 34 therethrough, as will also be discussed below in greater detail.

The second portion 222 can extend from the first portion 214 and can include ratchet teeth 258 along a longitudinal length of a first side 262 thereof. Ratchet teeth 258 can be configured to engage ratcheting pawl 266 (Figure 5) formed in hollow body 38 of outer handle body 18. Ratcheting pawl 266 can be biased into engagement with ratchet teeth 258 when ratcheting member 30 is positioned in an assembled configuration within outer handle body 18, as will be discussed in greater detail below. The tension release member 70 can be in selective engagement with ratcheting pawl 266. In this regard, movement of tension release member 70 relative to aperture 66 and ratcheting pawl 266 can selectively disengage ratcheting pawl 266 from ratchet teeth 258 to release any tension imparted on associated graft strands coupled to tensioning arms 114, as will also be discussed in greater detail below.

The second portion 222 can include a second side 274 opposite first side 262, as shown for example in Figure 10. Second side 274 can be configured to mate with an open end or side 278 of the C-shaped second portion 94 of inner handle body 22, as shown for example in Figure 9 with reference to Figure 6. Second side 274 can also include a longitudinally extending rib or protrusion configured to be received in the open end of the C-shaped second portion 222 to guide the ratcheting member 30 relative to inner handle body 22. In one exemplary configuration, the longitudinally extending protrusion can include an arcuate outer surface in cross-section configured to slidably engage an outer surface 286 (Figure 11) of driver shaft 34 when the driver shaft 34 is received in inner handle body 22 in an assembled configuration of tensioner 10.

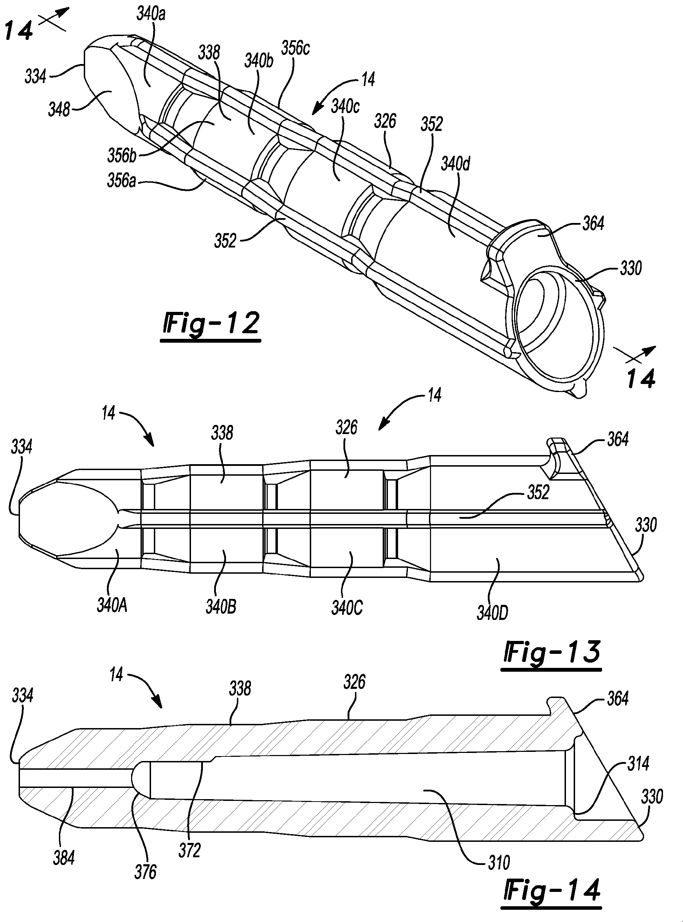

Driver shaft 34 can include a shaft body 294 extending from a proximal end 298 to a distal end 302, as shown for example in Figure 11. In the exemplary configuration illustrated, proximal end 298 can include an impact or driver receiving member 300 secured thereto or integrally formed thereon. Distal end 302 can be configured to receive implant 14 thereon, as will be discussed below in greater detail. Briefly, however, distal end 302 can include an implant receiving portion 306 configured to be received in a bore 310 (Figure 13) of implant 14. An open end of bore 310 can include a shoulder 314 configured to engage a corresponding shoulder 318 at a proximal end 322 of implant receiving portion 306. As will be discussed in greater detail below, driver shaft 34 can be impacted at proximal end 298 to drive implant 14 into a tibial tunnel, during which an impact force is transmitted from driver shaft 34 to implant 14 via engagement of corresponding shoulders 314, 318.

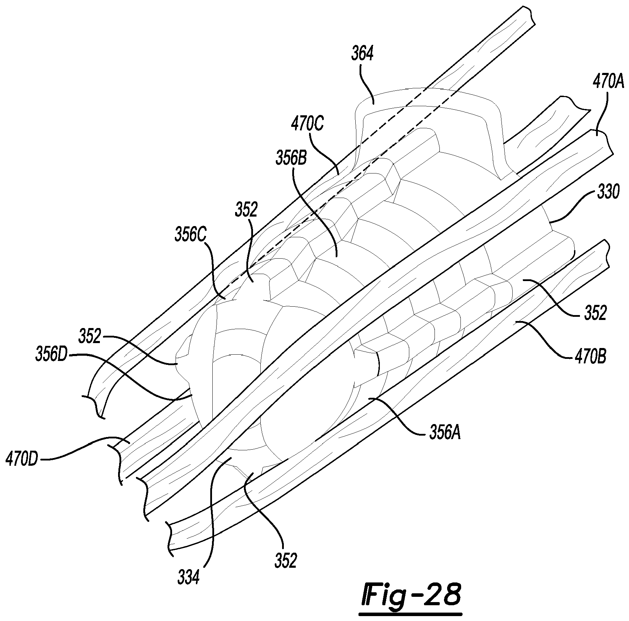

Implant 14 can include body 326 having an open proximal end 330, a closed or substantially closed distal end 334, and the closed or substantially closed end bore 310 extending from proximal end 330 toward distal end 334, as shown for example in Figures 12-14. Body 326 can include an outer surface 338 having a plurality of annular portions or segments 340 of increasing outer diameter in a direction from the distal end 334 toward the proximal end 330. In the exemplary configuration illustrated, implant 14 can include four segmented sections 340A-340D, each having an increasing diameter as shown in Figure 13. The distal end 334 can include a pair of opposed flattened sections 348 configured to aid in orientating the graft strands about implant 14, as will be discussed in greater detail below. In this regard, body 326 can also include four longitudinally and radially outwardly extending ribs 352 extending between the proximal and distal ends 330, 334 about outer surface 338. In the exemplary configuration illustrated, ribs 352 can be circumferentially spaced 90 degrees apart to divide outer surface 338 into four equally spaced quadrants 356A-356D configured to receive, in one exemplary configuration, four respective graft strands, as shown for example in Figure 28.

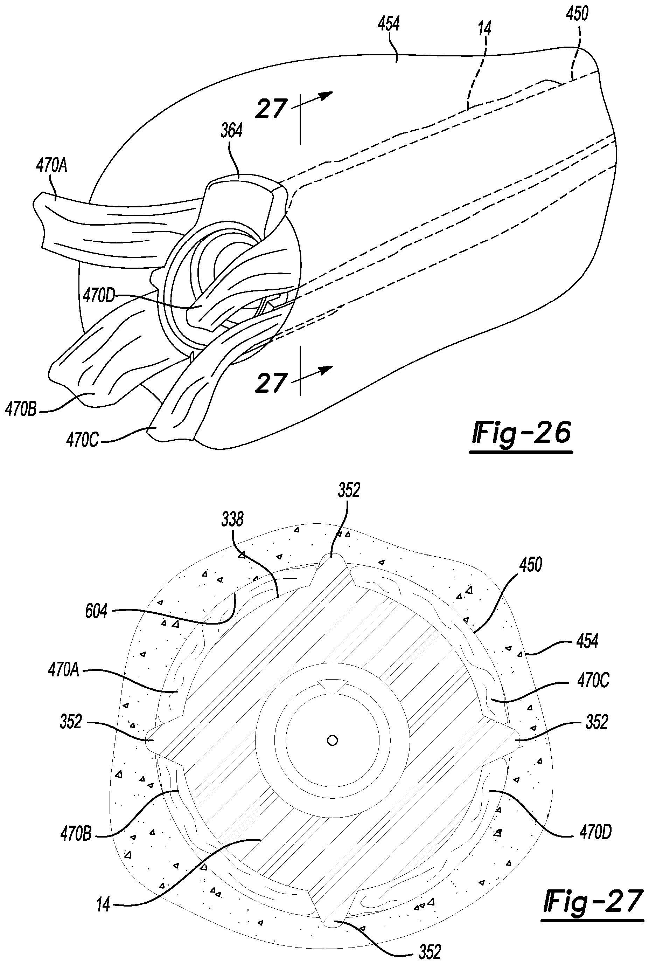

Proximal end 330 of implant 14 can include a 45 degree angle relative to a longitudinal axis of the implant so as to mate in a substantially flush or otherwise substantially parallel manner with an outer surface of a tibia when implanted into a tibial tunnel formed at an approximate 45 degree angle to a longitudinal axis of the tibia. A protrusion 364 can extend from the proximal end 330 and can be configured to engage the outer surface of the tibia 454 to provide a tactile feel to the surgeon indicative of the implant 14 being driven an appropriate distance into the tibia and to prevent implant 14 from being driven too deeply into the tunnel, as shown for example in Figure 26 with reference to Figure 12. Bore 310 of implant 14 can include a reduced diameter portion 372 at a closed or substantially closed end 376 thereof that is sized and shaped to provide an interference fit with an end of the implant receiving portion 306 of driver shaft 34. The interference fit can aid in retaining implant 14 in a desired orientation relative to driver shaft 34 and the tibial tunnel during an ACL reconstruction procedure, as will be discussed below in greater detail.

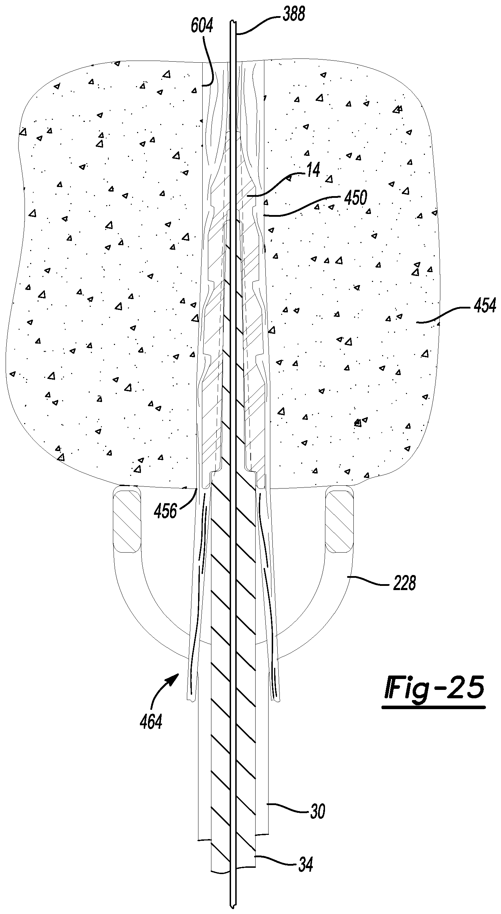

In one exemplary configuration, driver shaft 34 can include a throughbore or cannulation 380 and implant 14 can include a throughbore 384 at a distal end thereof such that the bores 380, 384 are in alignment when implant 14 is received on driver shaft 34, as shown for example in Figures 4, 11 and 14. As will be discussed in greater detail below, the throughbores 380, 384 can receive a guidewire 388 (Figure 25) for assisting in guiding implant 14 relative to the tibial tunnel 450.

With particular reference to Figures 1-4, an assembly configuration of tensioner 10 will now be discussed in greater detail. A spring 400 can be received over the second portion 94 of inner handle body 22 such that a proximal end 404 of spring 400 abuts flange 90. Inner handle body 22 can then be inserted into outer handle body 18 such that the distal end 82 of inner handle body 22 extends beyond the distal end 46 of outer handle body 18. Tension arm assembly 26 can be rotatably coupled to the distal end 46 of inner handle body 22 such that the recesses 124 of arm support member 110 engage the corresponding projections 130 of inner handle body 22. The second portion 94 of inner handle body 22 can include a length that cooperates with spring 400 such that a distal end 408 of spring 400 engages internal flange 102 of outer handle body 18 when tension arm assembly 26 is coupled to inner handle body 22, as discussed above. In this regard, spring 400 can exert a slight bias force against inner handle body 22, thereby urging inner handle body 22 proximally relative to outer handle body 18 until tension arm assembly 26 engages the distal end 46 of outer handle body 18.

In the exemplary configuration illustrated, one of the engagement surfaces 202, 206 of tensioning arms 114 can contact respective flanges 54 of outer handle body 18 under the bias force of spring 400 when the tensioner 10 is otherwise not in use. For example, when tensioning arms 114 are in the non-deployed or first position, as shown in Figure 1, engagement surface 206 of tensioning arm 114 can engage flanges 54 and the bias force of spring 400 in cooperation with the mating engagement of surfaces 202 and flanges 54 can maintain the arms in the non-deployed position. Similarly, tensioning arms 114 can be rotated to the deployed or second position, as shown for example in Figure 22, and can be maintained in this position by the bias force of spring 400 in cooperation with the mating engagement of surfaces 202 and flanges 54. For example, surface 202 can be orthogonal to surface 206, as shown in Figure 7, such that when one or both tensioning arms 114 are rotated ninety degrees from the first position to the second position, the bias force of spring 400 in cooperation with the mating engagement of surface 202 and flanges 54 can maintain the tensioning arms 114 in the deployed or second position.

Ratcheting member 30 can be received through tension arm assembly 26 and into outer handle body 18 about the open end of C-shaped second portion 94 of inner handle body 22, as briefly discussed above. Drive shaft 34 can be received through proximal end 78 of inner handle body 22 such that it extends into and through C-shaped second portion 94 and throughbore 244 of ratcheting member 30, as shown for example in Figures 1, 2 and 4. Implant 14 can be coupled to implant receiving portion 306 of driver shaft 34. It should be appreciated that the order or sequence of assembled components of tensioner 10 discussed above is merely exemplary and other assembly sequences are possible and contemplated herein.

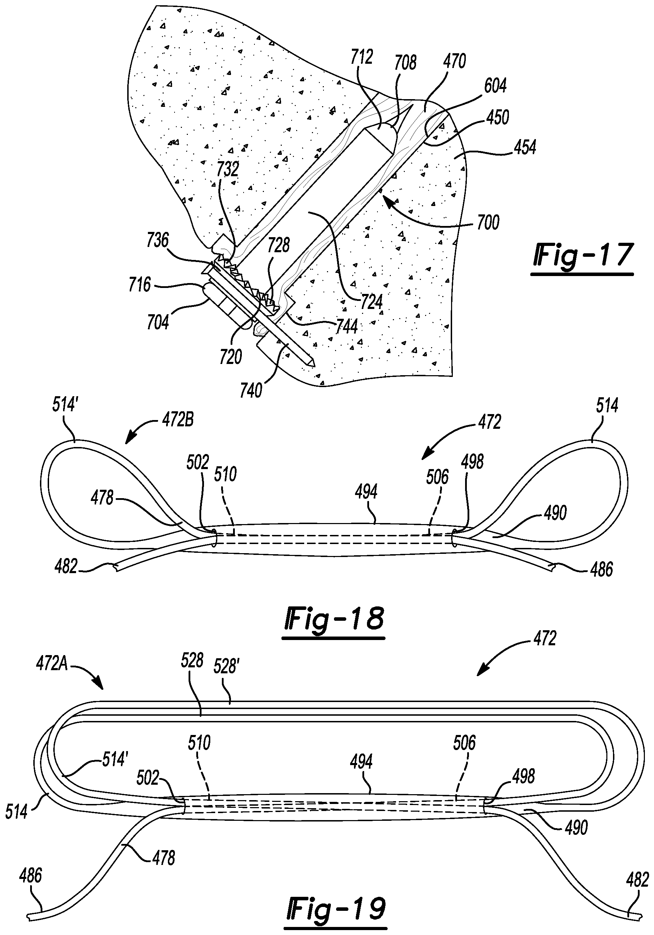

With additional reference to Figures 18-28, operation of tensioner 10 will now be discussed in greater detail in connection with an exemplary ACL reconstruction procedure. The exemplary ACL reconstruction procedure will make reference to a first or tibial tunnel 450 formed or drilled into a tibia 454 and a second or femoral tunnel 458 formed or drilled into a femur 462, as shown for example in Figure 20. The tibial tunnel 450 can include a first end or entrance 452 and a second end or exit 456 opposite of a joint space 460 between the femoral and tibial tunnels. Similarly, femoral tunnel 458 can include a first end or entrance 466 adjacent joint space 460 and can include a second end or exit 474, or can be formed as a blind tunnel. In one exemplary configuration, the tibial tunnel 450 can be formed at about a 45 degree angle to a longitudinal axis of the tibia, as is also known in the art. The tunnels 450, 458 can be formed in any appropriate manner, such as those generally known in the art, and will not be described in further detail herein.

Once the tunnels 450, 458 are formed, a natural or artificial graft loop or bundle 464 can be passed through the tibial tunnel 450 and into the femoral tunnel 458 as shown for example in Figures 20 and 21. In the exemplary configuration, the graft bundle 464 can include two graft loops 468 looped around an adjustable self-locking flexible member or suture construct 472 (Figures 18 and 19). The two graft loops 468 can thus include four graft strands 470 extending from an exemplary flexible member construct 472, as shown for example in Figure 20. It should be appreciated that while the discussion continues with reference to two graft loops and four graft strands, various other graft configurations can be used, including more or less graft loops and/or individual graft strands secured together at one end. In one exemplary aspect, graft 464 can include a synthetic or natural graft, such as an autograft or an allograft.

Adjustable self-locking flexible member construct 472 can include a double loop configuration 472A shown in Figure 19 or a bowtie loop configuration 472B shown in Figure 18. With particular reference to Figure 18, adjustable flexible member construct 472B can include a hollow flexible member or suture 478 having a first end 482 and a second end 486, and can include a body 490 that defines a longitudinal passage portion 494 therein between first and second ends 482, 486. The passage portion 494 can define a pair of apertures 498, 502 at opposed ends thereof. To form construct 472B, the first end 482 can be passed through aperture 498 and passage portion 494 and out aperture 502 such that a portion 506 of flexible member 478 following first end 482 extends through passage portion 494. In a similar manner, second end 486 can be passed through aperture 502 and passage portion 494 and out aperture 498 such that a portion 510 of flexible member 478 following second end 486 also extends through passage portion 494. This configuration forms two loops 514 and 514', as shown in Figure 18. It should be appreciated that each of the first and second ends 482, 486 can alternatively be pushed through a respective space defined between adjacent individual fibers of the braided flexible member 478 such that the respective spaces defined between fibers comprise apertures 498, 502 in communication with an interior longitudinal passage.

The pulling or tensioning of ends 482, 486 can cause movement of portions 506, 510 relative to passage portion 494, and the loops 514, 514' can be reduced to a desired size or placed in a desired tension. Tension in loops 514, 514' can cause the body 490 defining the passage portion 494 to be placed in tension and therefore cause passage portion 494 to constrict about portions 506, 510 passed therethrough. This constriction reduces the diameter of passage portion 494, thus forming a mechanical interface between the exterior surfaces of portions 506, 510 and an interior surface of passage portion 494. This constriction results in static friction between the interior and exterior surfaces at the mechanical interface, causing the adjustable flexible member 478 to "automatically" lock in a reduced size or diameter configuration in which tension is maintained. Flexible member construct 472B with adjustable loops 514, 514' can be used to position and tension a replacement graft, such as in an ACL reconstruction procedure, as will be discussed herein.

With reference to Figure 19 and continuing reference to Figure 18, adjustable flexible member construct 472A can be formed to include a double loop configuration having two loops 514, 514' that each traverse a path from one end of passage portion 494 to the other end thereof, instead of each loop being disposed at respective opposite ends of passage portion 494 as in construct 472B. Flexible member construct 472A can be formed by passing the first end 482 of the flexible member through aperture 502, through passage portion 494 and out aperture 498. The second end 486 can be passed through aperture 498, through the passage portion 494 and out the aperture 502. In various aspects, the first and second apertures 498, 502 can be formed during the braiding process as loose portions between pairs of fibers defining the flexible member 478, as discussed above. Passing ends 482, 486 through the apertures 498, 502 can form the loops 514, 514'. The loops 514, 514' can define mount or summit portions 528, 528' of the adjustable flexible member construct 472A and can be disposed generally opposite from the passage portion 494. Flexible member construct 472A with adjustable loops 514, 514' can be similarly used to position and tension a replacement graft, such as in the ACL reconstruction procedure.

The longitudinal and parallel placement of the first and second ends 482, 486 of the flexible member 478 within the passage portion 494 resists the reverse relative movement of the first and second portions 506, 510 of the flexible member construct 472 once it is tightened. The tensioning of the ends 482, 486 can cause relative translation of the portions 506, 510 relative to passage portion 494. Upon applying tension to the first and second ends 482, 486, the loops 514, 514' can be reduced to a desired size or placed in a desired tension. Tension in the loops 514, 514' can cause the body of the flexible member 478 defining the passage portion 494 to be placed in tension and therefore cause passage portion 494 to constrict about the portions 506, 510 similarly to the constriction discussed above with respect to construct 472B. This constriction can cause the adjustable flexible member construct 472A to "automatically" lock in a reduced size or smaller diameter configuration without the use of a knot. A further discussion of the flexible member constructs 472 is provided in U.S. Patent Serial No. 11/541,506 filed on September 29, 2006 entitled "Method and Apparatus for Forming a Self-Locking Adjustable Suture Loop" assigned to Biomet Sports Medicine, LLC, now Pat. No. 7,601,165.

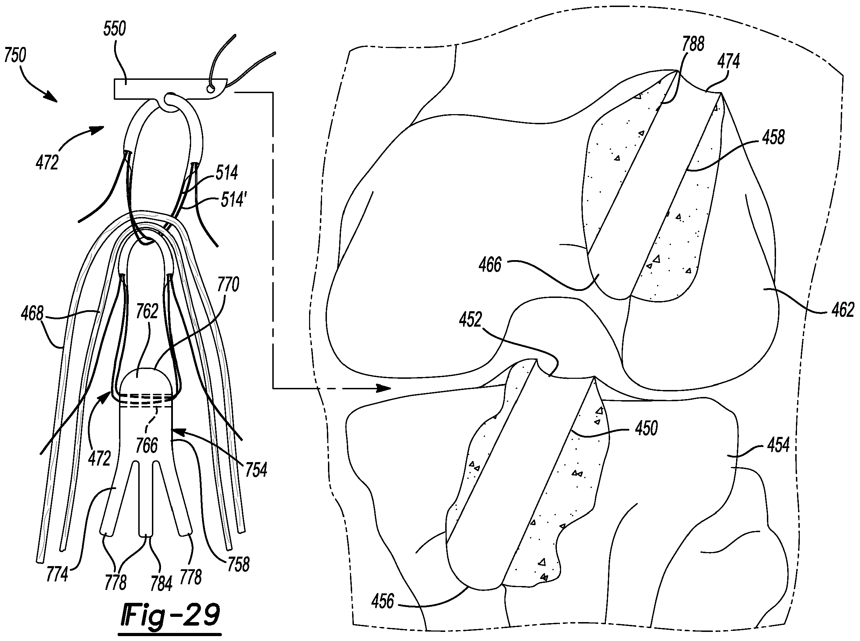

With reference to Figure 20, passage portion 494 of flexible member construct 472 can be coupled to a femoral fixation member, such as a fixation member 550, to facilitate femoral fixation or retention of graft 464 relative to femoral tunnel 458. The fixation member 550 can be, for example, a product sold by Biomet Sports Medicine, LLC under the name ToggleLoc™. A further discussion of the fixation member 550 can be found in U.S. Pat. No. 7,601,165. Fixation member 550 with construct 472 coupled thereto can be passed through the tibial and femoral tunnels 450, 458 and coupled to an outer surface 554 of femur 462, as shown in Figure 20. Fixation member 550 can be sized and shaped to include a first profile that allows insertion through the tibial and femoral tunnels 450, 458, and a second profile that allows engagement with positive locking surface upon rotation outside of the femoral tunnel 458. It should be appreciated that other apparatus and associated techniques can be used for femoral fixation in connection with an ACL reconstruction procedure, such as for example a femoral cross pin with the blind femoral tunnel and/or those discussed below in connection with one or more of Figures 29-40.

At this point, graft 464 can be passed through the loops of construct 472 extending from tibial tunnel 450. Tensioning of the first and second ends 482, 486 applies tension to the loops, thus pulling the graft 464 into the tibial and femoral tunnels 450, 458, as shown in Figure 21. In this regard, the first and second ends 482, 486 can extend through the tibial and femoral tunnels 450, 458 and then be tensioned. In this configuration, the ends 482, 486 can then be removed from the tibial tunnel 450 such that they exit through the joint space 460, as shown in Figure 21. Alternatively, the ends 482, 486 can extend only through the femoral tunnel 458 and then through the joint space 460 before they are subsequently tensioned. In the exemplary configuration shown in Figure 21, the loops of construct 472 can be fully tensioned to draw graft 464 up to passage portion 494. In an alternative exemplary configuration, the loops can be tensioned to draw graft 464 into the femoral tunnel and proximate passage portion 494, while stopping short of fully tensioning the loops to thereby provide for further tensioning of the loops after tibial fixation of the graft has occurred, as will be discussed below in greater detail.

With particular reference to Figures 22-27, tensioning and tibial fixation of graft 464 with tensioner 10 will now be discussed in greater detail. After coupling graft 464 to femur 462 with member 550, the four strands 470 can extend through and exit the tibial tunnel 450, as shown in Figure 22. At this point, tensioner 10 can be positioned or aligned with the tibial tunnel 450 such that the foot 228 of ratcheting member 30 is approximately parallel to an adjacent surface of the tibia, as also shown in Figure 22. As discussed above, the foot 228 can include a forty-five degree angle relative to the longitudinal axis 134 of tensioner 10 so as to align the tensioner longitudinal axis 134 with an axis of the tibial tunnel 450, which can also be formed at a forty-five degree angle relative to a longitudinal axis of the tibia 454. It should be appreciated that other angles can be used for the foot 28 and tunnel 450. In one exemplary configuration, the guidewire 388 can be inserted into the tibial tunnel such that one end extends into the joint space 460 and the other end extends from an opposite end 456 of the tibial tunnel 450. In this configuration, the tensioner 10 can be aligned with the tibia 454 such that the guidewire 388 is received into throughbore 380 of driver shaft 34. The guidewire 388 can include a predetermined length so as to not extend to the proximal end 298 of driver shaft 34.

The tensioning arms 114 can be rotated from the first non-deployed position shown in Figure 1 to the second deployed position shown in Figure 22 in anticipation of coupling the graft strands 470 thereto. It should be appreciated that tensioning arms 114 can be placed in the deployed position before or after aligning the tensioner 10 with the tibial tunnel 450. Similarly, the graft strands 470 can be coupled to the tensioning arms 114 before or after aligning tensioner 10 relative to tibial tunnel 450. Each graft strand 470 can be coupled to a respective attachment arrangement 166 at the longitudinal ends of each tensioning arm 114. In this regard, each arm can be coupled to two graft strands 470 via suture or flexible member 170 coupled to the respective graft strands, as shown in Figure 22. At this point, the tensioning arms 114 can be engaged with outer body flanges 54, as also shown in Figure 22.

To tension graft strands 470 a first time, the surgeon can apply a force to the outer handle body 18, such as by pulling with one hand against flange 50, to compress spring 400 between flanges 102 and 90 and move the outer handle body 18 relative to the inner handle body 22 and ratcheting member 30, as shown for example in Figure 23. As the tension arm assembly 26 is coupled to the inner handle body 22 and the graft strands 470, compressing spring 400 by moving outer handle body away from the tibia exerts a force on the inner handle body 22 toward the tibia thereby pushing the ratcheting member 30 incrementally toward the tibia 454. This action increases a longitudinal length of the overall tensioner 10 and thus applies an increasing amount of tension to graft strands 470 by moving tensioning arms 114 farther away from tibial tunnel 450. As outer handle body 18 is moved to apply tension to strands 470, ratchet pawl 266 engages the ratchet teeth 258 to maintain a desired position of outer handle body 18 relative to the ratcheting member 30 and thus maintain the desired tension. In addition, as tension is applied to the graft strands 470 with tensioner 10, bone spike 236 can engage the tibia 454 to aid in maintaining the position of tensioner 10 with tibial tunnel 450 during the ACL reconstruction procedure. In this regard, the tensioner 10 can stay in place relative to the tibial tunnel and tibia when under tension and thus does not require a user to hold or otherwise support tensioner 10 to maintain its position relative to the tibia when under tension.

The first portion 86 of inner handle body 22 can also include a scale 570 having indicia 574 indicative of an amount of tension being imparted onto graft strands 470 by operation of tensioner 10 as shown for example in Figures 9, 22 and 23. In this regard, spring 400 can be calibrated relative to scale indicia 574 such that as outer handle body 18 is moved relative to inner handle body 22 to tension the graft strands 470, the flange 50 can align with a particular indicia 574 to indicate an amount of tension being placed on graft strands 470.

As tension is applied to graft strands 470 as discussed above, tension arm assembly 26 moves away from the distal end of outer handle body 18, as shown in Figure 23. At this point, arm support member 110 can now rotate about axis 162 relative to inner handle body 22, as also shown in Figure 23. Allowing arm support member 110 to rotate relative to inner handle body 22 in cooperation with allowing tensioning arms 114 to individually rotate about axis 160 relative to arm support member 110 provides for being able to apply equal tension to each of graft strands 470. In this regard, the rotating capabilities of arm support member 110 and arms tensioning 114 can compensate for differences in length of individual graft strands 470 and/or the accompanying attachment sutures to balance the tension in each graft strand. With this compensation capability, the surgeon or clinician does not have to ensure that each graft strand 470 and accompanying suture exiting the tibial tunnel 450 has the same length from the tunnel 450 to the tensioner 10.

Once tension is applied to the graft strands 470 as discussed above, the tensioner 10 can maintain the tension of strands 470 until released via actuation of tension release member 70. In this regard, upon tensioning graft strands 470 with tensioner 10, the tensioner can remain secured to the tibia 454 and aligned with the tibial tunnel 450 without the aid of the surgeon or clinician continuing to hold the tensioner 10. With this capability of tensioner 10, the surgeon can, for example, cycle the knee joint to remove any laxity from the tensioned graft while the tensioner essentially self-maintains its position relative to the tibia 454 and tibial tunnel 450. If such cycling of the joint results in a decrease of tension on the graft strands 470, the surgeon can again pull or move outer handle body relative to inner handle body to tension the graft strands 470 a second time to increase the tension on strands 470 to the desired amount.

With the desired tension being applied to the graft strands by tensioner 10 and the knee joint being optionally cycled as discussed above, the implant 14 can be implanted into the tibial tunnel 450 to secure the tensioned graft strands 470 to the tibia 454, as will be discussed in greater detail below. It should be appreciated that by securing the grafts strands 470 about the horizontally and vertically spaced apart four attachment arrangements 166 of the tension arm assembly 26, the graft strands 470 are effectively separated into four quadrants at the exit of the tibial tunnel 450 corresponding to the four quadrants 356A-356D of implant 14 as shown for example in illustrative Figure 28.

Implant 14 can then be advanced by translating driver shaft 34 relative to the inner and outer handle bodies 18, 22 and the ratcheting member 30 such that implant 14 is proximate the entrance of tibial tunnel 450. The opposed flattened sections 348 at the distal end of implant 14 can aid in separating the graft strands 470 upon implantation such that two strands 470A, 470B are on one side of the implant 14 and the other two strands 470C and 470D are on the opposite side of the implant 14 as shown for example in illustrative Figure 28. The longitudinal ribs 352 can aid in further separating the strands 470 upon implantation such that each strand 470A-470D is positioned in a respective quadrant 356A-356D. It should be appreciated that while strands 470A-470D have been discussed above as correlating to quadrants 356A-356D, strands 470A-470D can be placed in different quadrants 356A-356D so long as one strand is in each quadrant 356A-356D.

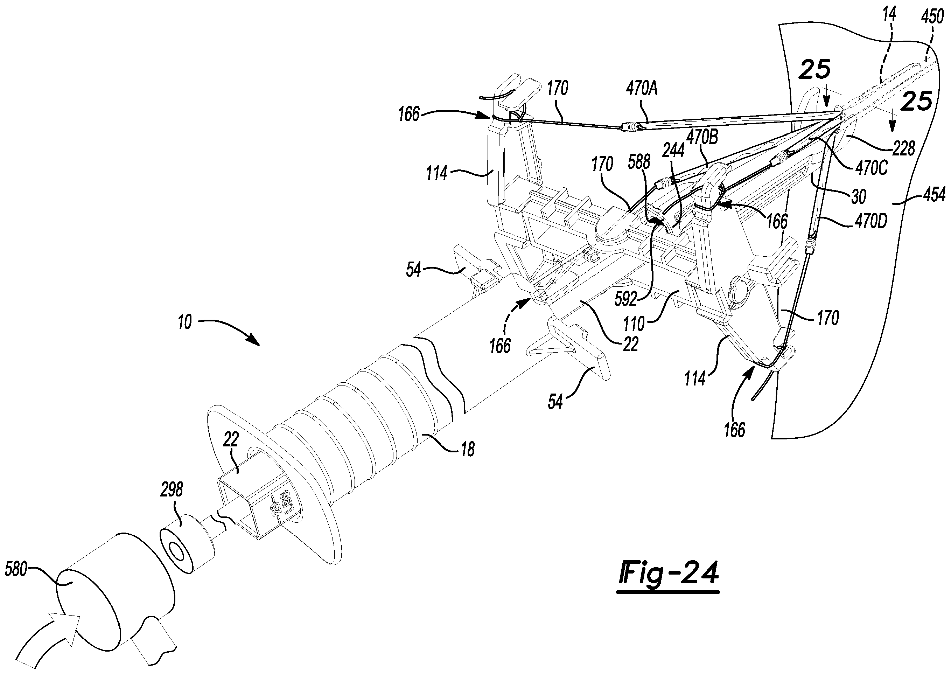

A driving or impacting instrument 580 can be used to impart a driving force on the proximal end 298 and translate driver shaft 34 relative to the outer and inner handle bodies 18, 22 and ratcheting member 30 along the axis of the tibial tunnel 450. Such translation of driver shaft 34 can drive implant 14 into tibial bone tunnel 450 about graft strands 470A-470D to fix graft 464 relative to the tibia 454, as shown in Figure 24 with reference to Figures 25-27. Implant 14 can be driven axially into the tibial tunnel 450 until protrusion 364 extending from the proximal end 330 thereof engages an adjacent outer surface of the tibia 454. Protrusion 364 can serve as a stop and/or provide a tactile feel to the surgeon that implant 14 has been driven to the desired depth. In an exemplary configuration, driver shaft 34 can also include indicia, such as an indicator band 588, to provide an additional indication of the desired drive depth. In this configuration, the indicator band 588 can be adjacent a proximal end 592 of the throughbore 244, as shown for example in Figure 24.

As the implant 14 is driven into the tibial bone tunnel, implant 14 can compress the graft strands between an outer surface 338 of implant 14 and an inner surface or wall 604 of the tibial tunnel 450, as shown for example in Figures 25 and 27. In one exemplary configuration shown in Figure 25, implant 14 can compress each graft strand 470A-470D to substantially fill an area between adjacent longitudinal ribs 352 and the inner surface 604 of the tibial tunnel 450 and the outer surface 338 of implant 14. In this regard, implant 14 can include an increasing outer diameter with each segmented section 340A-340D, as discussed above, where the first segment 340A includes an outer diameter less than an inner diameter of wall 604 of tibial bone tunnel 450, and the last segmented section 340D can include a diameter greater than the inner diameter of wall 604.

In one exemplary configuration and with reference back to Figures 12 and 13, each segment can include an inner diameter increasing by approximately 0.5 mm. In this regard, the first segment can include an outer diameter 0.5 mm less than the inner diameter of wall 604, and the last segment has an outer diameter 1.0 mm greater than the inner diameter of wall 604. This configuration can provide an interference fit with the cancellous bone of the tibia 454 where the cancellous bone is compressed outward by the implant 14 to provide tibial fixation of graft strands 470, as discussed above.

Upon driving implant 14 into tibial tunnel 450 to provide the tibial fixation of graft strands 470, any graft strands extending out from the tibial tunnel 450 and implant 14 can be trimmed and removed. The optional guidewire 388, if used, is also removed. Tensioner 10 will then no longer be coupled to the tibia 454 and can be subsequently removed. In one exemplary configuration, the loops of construct 472 can be tensioned to draw graft 464 into the femoral tunnel and proximate passage portion 494, while stopping short of fully tensioning the loops to thereby provide for further tensioning of the loops after tibial fixation of the graft has occurred, as discussed above. In this configuration, the graft 464 can be tensioned a third time, if desired, after removal of tensioner 10 by further tensioning the first and second ends 482, 486 extending from the joint space (Figure 21). Tensioning the ends 482, 486 further reduces the size of the loops and thus applies additional tension to the graft strands 470 that are now fixed to tibia 454 after the first and second tensions. After tensioning is completed, excess ends 482, 486 extending from tibial tunnel 450 can be removed.

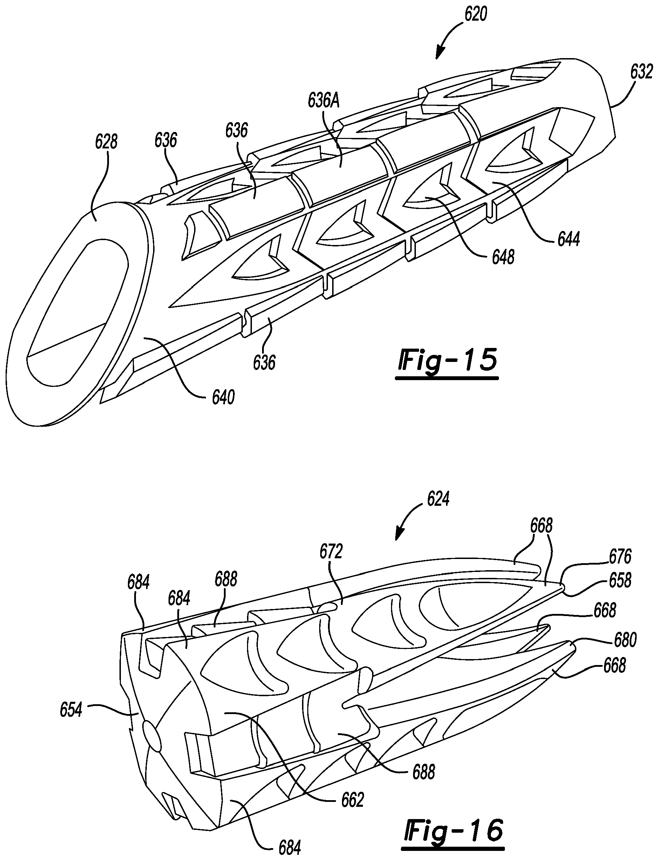

Turning now to Figures 15-16, alternative implants 620 and 624 will now be discussed according to various aspects of the present teachings. With particular reference to Figure 15, implant 620 can include an open proximal end 628 and a closed or substantially closed distal end 632 similar to implant 14. Implant 620 can also be configured for use with tensioner 10 in a similar manner as implant 14. As can be seen in Figure 15, proximal end 628 can be angled at 45 degrees relative to a longitudinal axis of implant 620 so as to be substantially parallel or parallel to the tibia 454 upon implantation. Implant 620 can include four longitudinal extending rib configurations 636 to separate an outer surface 640 into four circumferential quadrants, similar to implant 14 discussed above. In the exemplary configuration illustrated, each rib configuration 636 can include a plurality of segments 636A.

The outer surface 640 between adjacent rib configurations 636 can include a concave shape 644 in cross section and can include a plurality of graft engaging features 648. In the exemplary configuration illustrated, the graft engaging features 648 can include various depressions and/or projections configured to engage the graft strands 470. Implant 620 can also include an increasing outer diameter or dimension from the distal end 632 toward the proximal end 628. In one exemplary configuration, the distal end 632 can include an outer diameter less than the inner diameter of wall 604 of the tibial tunnel 450 and the proximal end 628 can include an outer diameter greater than the inner diameter of wall 604.

With particular reference to Figure 16, implant 624 can include a proximal end 654 and a distal end 658, and can also be configured for use with tensioner 10 in a manner similar to implant 14. Implant 624 can include a body 662 extending from the proximal end 654 toward the distal end 658 that transitions into four axially extending and circumferentially spaced apart leg members 668. Each leg member 668 can include a proximal end 672 having a width greater than a distal end 676, as shown in Figure 16. In the exemplary configuration illustrated, the distal ends 676 of each leg member can terminate in a distal tip or point 680. The body 662 can include four longitudinally extending protrusions 684 each axially aligned with a respective leg member 668, as shown in Figure 16. In this regard, each graft strand 470 can be positioned in the space between each leg member 668 and the corresponding recessed area 688 between each protrusion 684.

Turning now to Figure 17, another alternative implant 700 is illustrated for use in tibial fixation of graft strands 470. In one exemplary configuration, implant 700 can be used in conjunction with tensioner 10, where tensioner 10 is used to tension graft strands 470 in the manner discussed above. Implant 700 can be used to compress the graft strands 470 between the implant 700 and the inner diameter of wall 604 of tibial tunnel 450. Implant 700 can include a proximal end 704 and a distal end 708 terminating in a distal tip 712. Proximal end 704 can include a head member 716 configured to receive an impact or driving force to drive implant 700 into tibial tunnel 450 about graft strands 470, as shown for example in Figure 17. A graft engaging member 720 can be positioned adjacent head member 716 about a body 724 of implant 700 and can include a graft engaging surface 728. Graft engaging surface 728 can include a plurality of teeth or projections 732 configured to engage graft strands 470 and aid in fixing strands 470 to tibia 454.

Implant 700 can also include an aperture 736 extending through the implant proximate the head member 716 and transverse to a longitudinal axis of implant 700. In the exemplary configuration illustrated in Figure 17, aperture 736 can be positioned between or substantially between head member 716 and graft engaging member 720, and can receive a fastener 740 in securing implant 700 to the tibia 454. A counterbore or countersink 744 can be formed in the tibia 454 at the entrance of tibial tunnel 450 to receive the graft engaging member 720, as also shown in Figure 17. In one exemplary configuration, the body 724 of implant 700 can include a diameter or width that forms an interference fit relative to the inner wall of the tibial tunnel 450.

Turning now to Figures 29-30, a femoral fixation arrangement and associated technique will now be described. With particular reference to Figure 29, a flexible member construct assembly 750 is shown and can include a first flexible member construct 472 coupled to fixation member 550 in the manner discussed above. A pair of graft bundles 468 can also be looped over construct 472 in the manner discussed above. In addition, a second flexible member construct 472 can be coupled to the loops 514, 514' and to a graft fixation member 754, as shown in Figure 29.

Graft fixation member 754 can include a body 758 having a first portion 762 defining an aperture 766 adjacent a proximal end 770 thereof, and a second portion 774 having a plurality of leg members, such as four leg members 778, extending to and defining a distal end 784 of fixation member 754. Aperture 766 can receive a portion of the second flexible member construct 472 to couple fixation member 754 to the first flexible member construct 472, as discussed above and shown in Figure 29. The first portion 762 of body 758 can include a diameter or width substantially equal to or greater than an inner wall 788 of femoral tunnel 458 so as to be received in femoral tunnel 458 about graft strands 470 in an interference fit relationship. Alternatively, first portion 762 can include a diameter less than inner wall 788 so as to be received about graft strands 470 in a non-interference fit manner relying on leg members 778 for the femoral fixation, as will be discussed below. The leg members 778 can be biased radially outward from first portion 762 so as to have an increasing diameter or width in a direction toward distal end 784, as shown in Figure 29. Graft fixation member 754 can include any number of legs 778, such as three or four legs to secure and separate the four grafts strands 470.

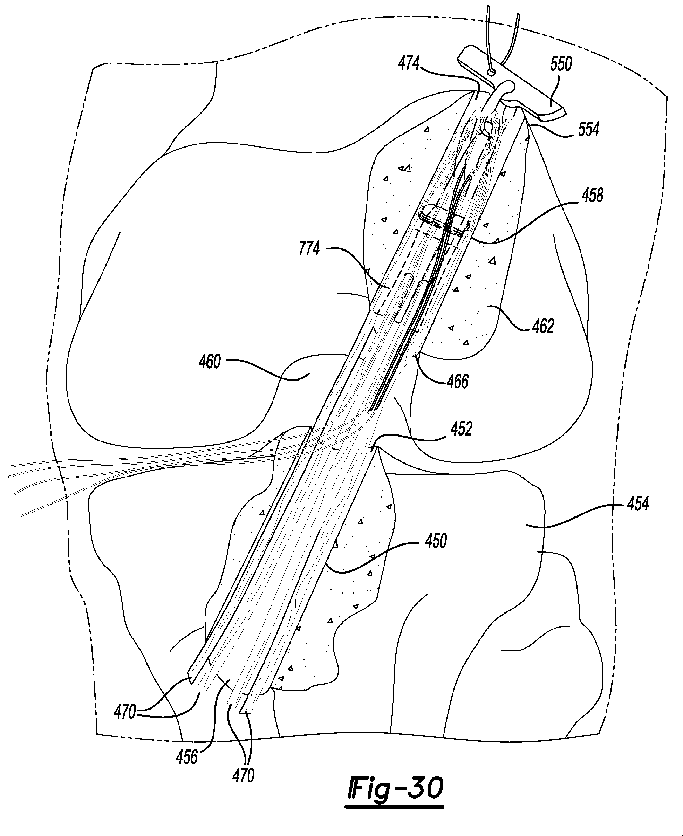

In use, and with reference to Figure 30, the fixation member 550 can be drawn through the joint space 460 and up through the femoral tunnel 458 such that the fixation member 550 engages the outer surface 554 of the femur 462 at exit 474 in the manner discussed above. The free ends of the first flexible construct 472 can be tensioned to draw the graft into the femoral tunnel 458 relative to fixation member 550, as shown in Figure 30 and discussed above with reference to Figure 21. At this point, the graft fixation member 754 can remain outside of the femoral tunnel adjacent the joint space 460 and near the opening 466. The free ends 482, 486 of the first and second flexible member constructs 472 can extend from the femoral tunnel out through the joint space 460 and/or can extend through the femoral and tibial tunnels 458, 450. In one exemplary configuration, the free ends of the first and second flexible member constructs can both extend out of the joint space 460 to provide for tensioning/drawing the graft strands 470 and/or the graft fixation member 754 after implant 14 or another tibial fixation member is implanted into tibial tunnel 450. The graft strands 470 can then be fed into and through the tibial tunnel 450 starting from the joint space side. Once the graft strands 470 are fed though the tibial tunnel 450, the grafts strands 470 can be tensioned using tensioner 10 in the manner discussed above or another appropriate tensioning arrangement.

Having tensioned graft strands 470, the free ends of the second flexible construct 472 can be tensioned to draw the graft fixation member 754 into the femoral tunnel 458 via the joint space 460. As the graft fixation member 754 is drawn into the femoral tunnel 458, the leg members 778 can be compressed against their biased outward position thereby exerting a force on the inner wall 788 of femoral tunnel 458 to provide femoral fixation and separation of the graft strands 470. In one exemplary configuration, the graft fixation member 754 can be drawn into the femoral tunnel 458 such that the distal end 784 of the graft fixation member 754 is positioned at the entrance 466 of the femoral tunnel 458 to provide the femoral fixation of graft strands 470 at the location where the graft extends from the femoral tunnel 458 into the joint space 460. This configuration can prevent movement of the graft strands 470 at the entrance 466 between flexion and extension and thereby increase wear resistance and stability of the graft 464.

The graft strands 470 can then be fixed to the tibia 454 using the aperture fixation technique discussed above in connection with tensioner 10 or another tibial tensioning and fixation technique. Once the graft strands 470 are fixed to the tibia 454, the free ends of the first flexible member construct 472 can optionally be tensioned to reduce the size of loops 514, 514' and thereby add additional tension to the graft strands 470 after tibial fixation. By having the free ends 482, 486 of the first and second flexible member constructs 472 extend from the femoral tunnel 458 out through the joint space 460, tensioning of the first and/or second flexible member constructs 472 can be accomplished after tibial fixation of the graft strands 470, which can obstruct the tibial tunnel 450.

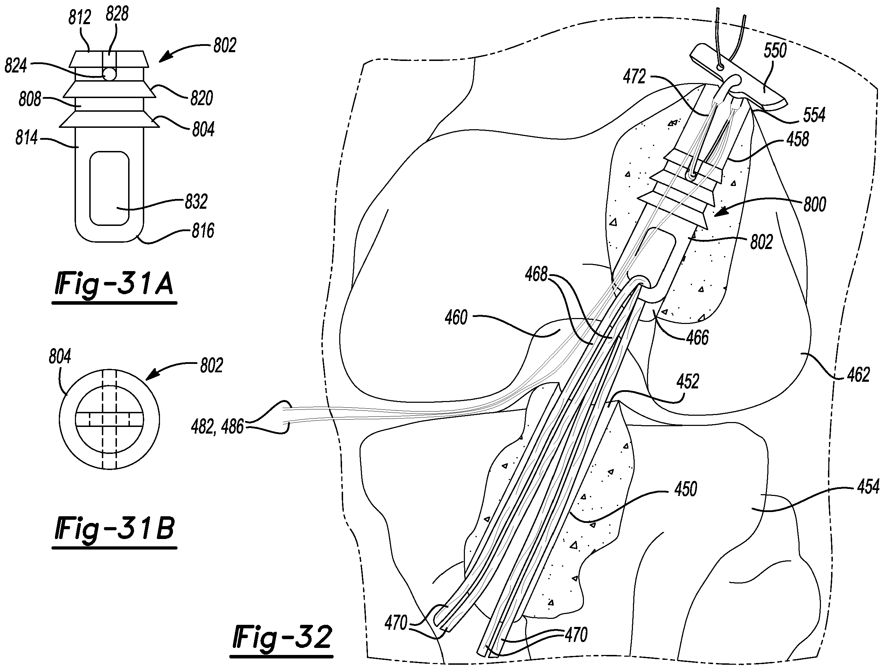

With additional reference to Figures 31A-32, another femoral fixation member and associated technique will now be discussed. A flexible member construct assembly 800 is shown and can include a first flexible member construct 472, fixation member 550, and a fixation member 802. Fixation member 802 can include a body 804 having a first portion 808 defining a proximal end 812 and a second portion 814 extending therefrom and defining a distal end 816. The first portion 808 can include annular ribs or barbs 820 sized and shaped to provide for movement in one axial direction, such as into the femoral tunnel 458, and to resist movement in an opposite direction as shown in Figure 31A. The first portion 808 can include a transverse aperture 824 adjacent the proximal end 812 that is configured to receive the loops 514, 514' of flexible member construct 472 to couple fixation member 802 thereto. A pair of notches 828 can be provided on opposite lateral sides of the first portion 808 in alignment with the aperture 824 for receiving a portion of the flexible member construct 472 therein.

The second portion 814 can define an opening 832 configured to receive graft bundles 468, as shown in Figure 32. Fixation member 550 of flexible member construct assembly 800 can be drawn into the femoral tunnel 458 from the joint space 460 or up through the tibial tunnel 450 and can be fixed to the outer surface 554 of the femur 462 in the manner discussed above. The free ends 482, 486 of the flexible member construct 472 can then be tensioned to draw the fixation member 802 into the femoral tunnel 458 such that the distal end 816 of fixation member 802 is at the entrance 466 adjacent the joint space 460. This configuration can thus also provide femoral fixation of the graft strands 470 at the entrance 466 and exit 474 of the femoral tunnel 458. The graft strands 470 can be tensioned using tensioner 10 or another suitable tensioning technique and can be fixed to the tibia using implant 14 or another suitable implant, such as those discussed herein.

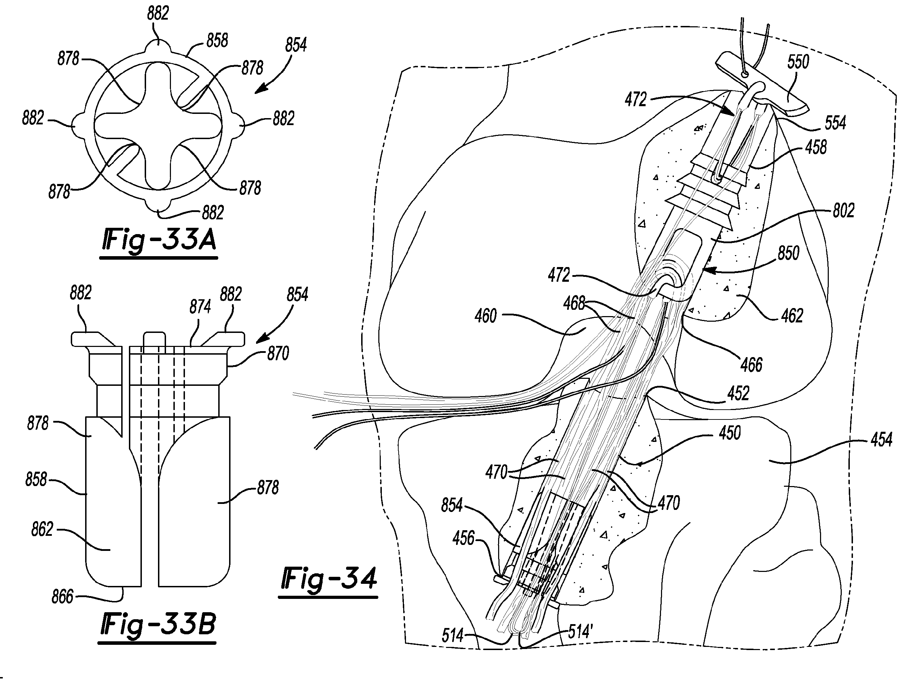

With additional reference to Figures 33A-34, another flexible member construct assembly 850 is shown. Flexible member construct assembly 850 can also include the first flexible member construct 472, fixation member 550 and fixation member 802 of assembly 800 discussed above. A second flexible member construct 472 can be coupled to opening 832 along with graft bundles 468, as shown for example in Figure 34. A tibial fixation member 854 can be used with assembly 850 in place of implant 14. As will be discussed below, fixation member 854 can be separate from flexible construct 472 such that it can be placed inside loops 514, 514' at an appropriate time during the procedure.

Fixation member 854 can include a body 858 having a first portion 862 defining a proximal end 866 and a second portion 870 extending therefrom and defining a distal end 874. First portion 862 can include an outer diameter or width sized to provide an interference fit with tibial tunnel 450 so as to provide aperture fixation of graft strands 470 when implanted in tibial tunnel 450 about graft strands 470. First portion 862 can include four axially extending concave portions 878 to receive graft strands 470 similar to the quadrants 356. The second portion 870 can include a plurality of radially outwardly extending tabs or projections 882 at the distal end 874 to provide a positive stop for fixation member 854 relative to tibial tunnel 450. In this regard, projections 882 can include an outer diameter or dimension greater than the inner diameter of tibial tunnel 450.

In use, fixation member 854 of assembly 850 can be drawn into the femoral tunnel 458 and positioned on the outer surface of the femur 462 in a manner similar to that discussed above in connection with assembly 800. If fixation member 802 is fed though joint space 460, then graft strands 470 and the loops and free ends of the second flexible construct 472 can be fed into and through the tibial tunnel 450. The free ends of the first flexible construct 472 can be tensioned to draw the fixation member 802 and graft strands 470 into the femoral tunnel 458. As discussed above, the free ends can be tensioned so as to position the distal end of fixation member 802 adjacent the exit 466 of femoral tunnel 458, as shown in Figure 34. The grafts strands 470 extending through the tibial tunnel can then be tensioned using tensioner 10 or with another technique, and the fixation member 854 can then be implanted into tibial tunnel 450 about graft strands 470 to provide tibial fixation of strands 470. To implant fixation member 854 into tibial tunnel 450, the loops 514, 514' extending from tibial tunnel 450 can be looped around fixation member 854 and then tensioned to draw fixation member into tibial tunnel 450 about graft strands 470 to fix strands 470 to the tibia 454. Fixation member 854 can be drawn into tibial tunnel 450 until projections 882 engage an outer surface of the tibia adjacent tunnel 450, as shown in Figure 34.

In an exemplary alternative configuration, the first flexible construct 472 can be tensioned to draw fixation member 802 partially into the femoral tunnel 458, but short of the desired position so as to provide an ability to further draw fixation member into the femoral tunnel 458 after tibial fixation of strands 470, as will be discussed below. The fixation member 854 can then be positioned in loops 514, 514' and pulled into the tibial tunnel to fix graft strands 470 to the tibia in the manner discussed immediately above. After tibial fixation of strands 470, the first flexible construct 472 can be further tensioned to further draw fixation member 802 into the desired position in the femoral tunnel 458 and at the same time tension graft strands 470 to the desired tension.

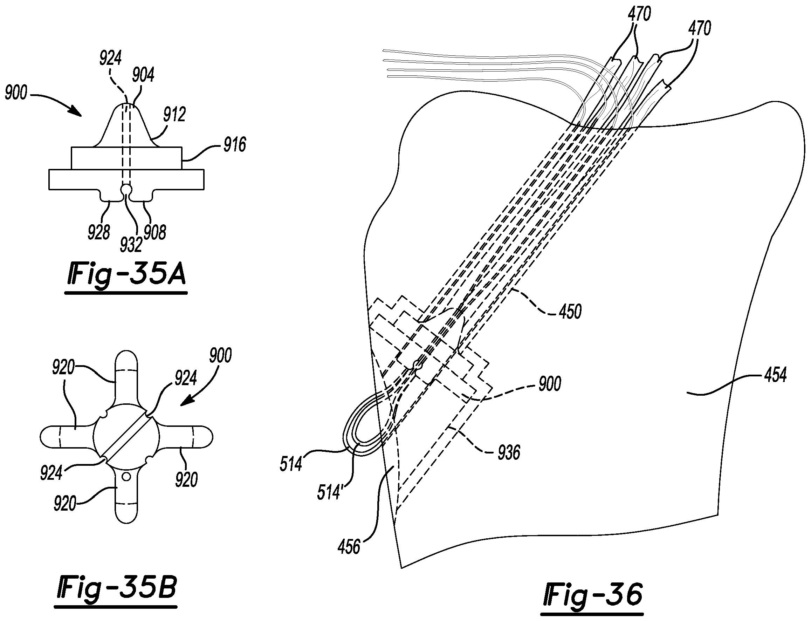

Turning now to Figures 35A-36, another tibial fixation member 900 is shown. Tibial fixation member 900 can include a proximal end 904, a distal end 908 and a body 912 extending therebetween. Body 912 can include a stepped configuration 916 having four radially outward extending members 920 forming four quadrants for graft strands 470. A notch 924 can extend along opposite lateral side of the fixation member 900 for receipt of a portion of flexible member construct 472, as will be discussed below in greater detail. Distal end 908 can include a projection 928 having a notch 932 formed therein and configured to receive suture loops 514, 514'.

In use, fixation member 900 can be placed in loops 514, 514' extending from tibial tunnel 450 and drawn into a counterbore 936 formed in the tibia 454 to fix graft strands 470 to tibia 454, as shown for example in Figure 34. In this regard, fixation member 900 can be used as an alternative to fixation member 802 with assembly 800 to effect tibial fixation of graft strands 470. Fixation member 900 can also be used as an alternative to fixation member 700 discussed above in connection with Figure 17. It should be appreciated that fixation members 854 and 900 can also be used as an alternative to fixation member 754 discussed above in connection with Figures 29 and 30. In this regard, second flexible member construct 472 of construct assembly 750 can be looped over one of fixation members 854 or 900 in place of being coupled to fixation member 754.

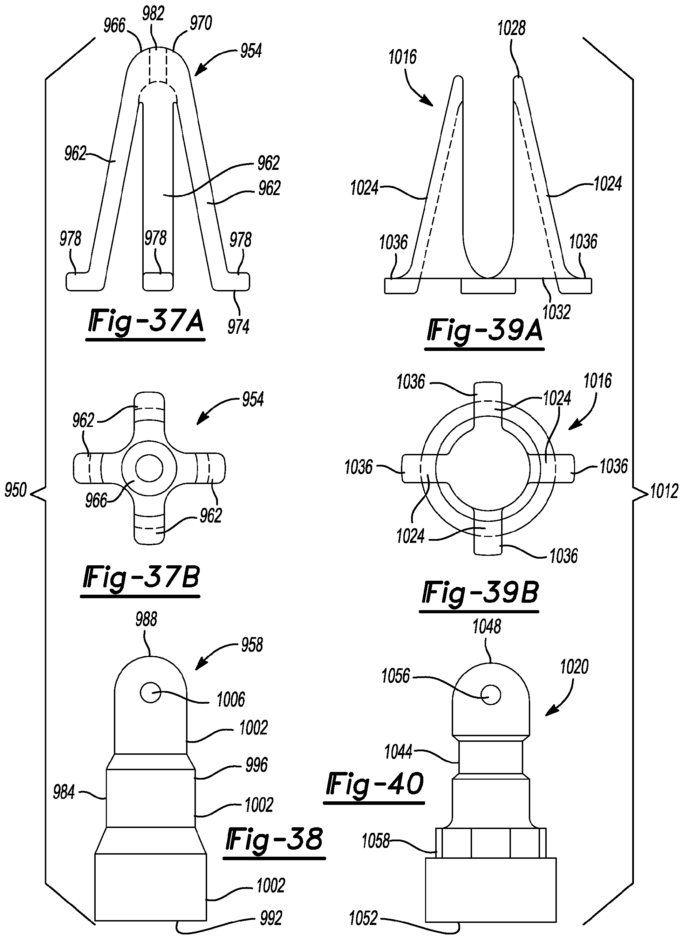

Turning now to Figures 37A-40, a pair of alternative graft fixation members will now be discussed. With particular reference to Figures 37A-38, a graft fixation assembly 950 is shown and can include an outer sheath member 954 and an inner or plug member 958. Fixation assembly 950 can be used for either femoral or tibial fixation of graft strands 470, as will be discussed below. Sheath member 954 can include four legs 962 extending from a common tip 966. Each leg member can increasingly extend radially outward from a proximal end 970 to a distal end 974. The legs 962 can include an outer diameter or dimension greater than a corresponding inner diameter of the tibial or femoral tunnels. Each leg 962 can include a projection 978 extending radially outward therefrom and configured to act as a positive stop when the sheath 954 is positioned in the femoral or tibial tunnels.

Plug member 958 can include a body 984 having a proximal end 988 and a distal end 992. Body 984 can include a stepped configuration 996 defining a plurality of segments 1002 having an increasing diameter from the proximal end 988 to the distal end 992, as shown in Figure 38. Plug member 958 can be received in the sheath member 954 inside of legs 962 so as to expend legs 962 outward between the four graft strands 470 and against a wall of the femoral or tibial tunnels. In this regard, the diameter of the distal end of plug member 958 can be sized to create an interference fit with the femoral or tibial tunnels when received in the sheath member 954 that is positioned in one of the femoral or tibial tunnels. The proximal end 988 of plug member 958 can include an aperture 1006 formed therein and configured to receive loops of flexible member construct 472, as will be discussed below.

In use, the loops of the first or second flexible member construct 472 (depending on whether the fixation assembly 950 will be used for femoral or tibial fixation) can be passed through an aperture 982 in the sheath member 954 and coupled to plug member 958 via aperture 1006. For example, and with reference back to Figure 29, fixation assembly 950 can be used in place of fixation member 754 where sheath member 954 and plug member 958 are coupled to the first flexible member construct 472. Alternatively, in another exemplary configuration and with reference back to Figure 34, fixation assembly 950 can be used for tibial fixation where sheath member 954 and plug member 958 are coupled to the second flexible member construct 472 of assembly 850 in place of fixation member 854.

In either femoral or tibial fixation, the sheath member 954 can be positioned in the respective bone tunnel between graft strands 470 by sliding the sheath member 954 about the loops 514, 514' until the projections 978 abut an outer surface of the respective tunnel similar to fixation member 854 shown in Figure 34. The corresponding flexible member construct 472 can then be tensioned to draw plug member 958 into sheath member 954 thereby expanding legs 962 into compressed engagement with surrounding bone of the bone tunnel to separate and fix graft strands 470 to the respective femur or tibia.

With additional reference to Figures 39A-40, graft fixation assembly 1012 will now be discussed. Graft fixation assembly 1012 is similar to graft fixation assembly 950 and can also be used for either femoral or tibial graft fixation in a similar manner as assembly 950. Graft fixation assembly 1012 can include an outer sheath member 1016 and a plug member 1020 configured to be received in the sheath member 1016, as will be discussed below. Sheath member 1016 can include four leg members 1024 extending from an open proximal end 1028 to an open distal end 1032, as shown in Figures 39A and 39B. Each leg member 1024 can extend radially outward as it extends from the proximal end 1028 to the distal end 1032 such that sheath member 1016 includes an increasing diameter or width from the proximal end 1028 to the distal end 1032, as shown in Figure 39A. In one exemplary configuration, the diameter at the distal end can be larger than an inner diameter of the femoral or tibial tunnel so as to create an interference fit when the sheath member is positioned therein. Each leg member 1024 can include a radially outward projection 1036 configured to serve as a positive stop in a similar manner as projections 978 of fixation assembly 950.

Plug member 1020 can also be similar to plug member 958 and can include a body 1044 having a proximal end 1048 and a distal end 1052. Proximal end 1048 can define an aperture 1056 for receiving flexible member construct 472 and the body can include an increasing diameter from the proximal end 1048 to the distal end 1052. Similar to plug member 958, a diameter of the distal end 1052 of plug member can be greater than a diameter of the proximal end 1028 of legs 1024 so as to expand the legs into compressive engagement with graft strands 470 and surrounding bone of the femur or tibia. Plug member 1020 can also include notches or depressions 1058 configured to engage the leg members 1024 and prevent rotation of plug member 1020 relative to sheath member 1016.

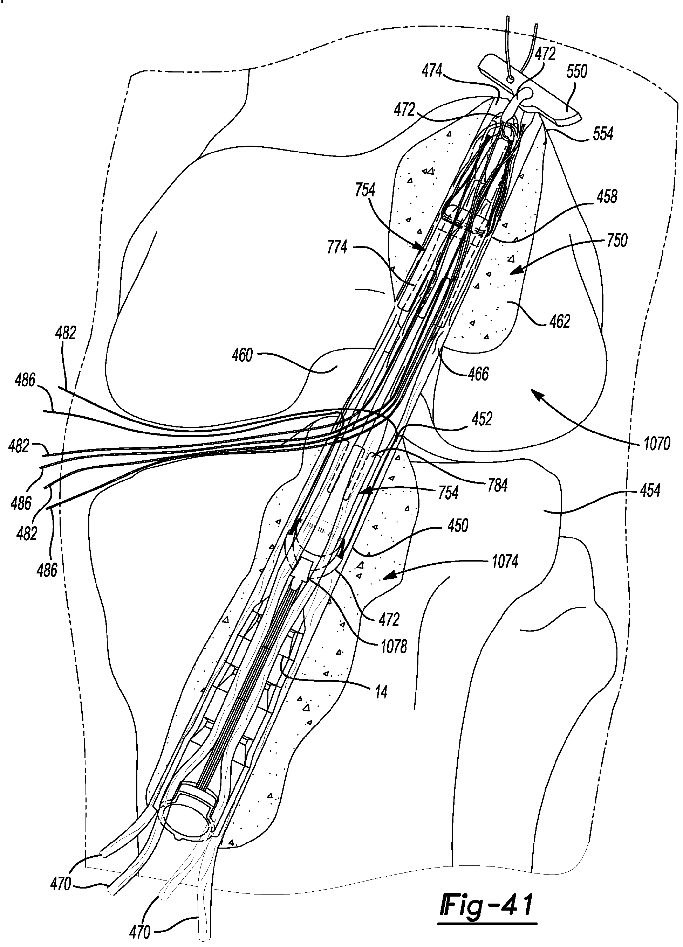

With additional reference to Figure 41, another flexible member construct assembly 1070 is shown. As will be discussed in greater detail below, construct assembly 1070 can be used to provide femoral and tibial fixation at the entrance and exit of the tibial tunnel 450 and the femoral tunnel 458. Construct assembly 1070 can include flexible member construct assembly 750 for use in femoral fixation in the manner discussed above and as shown in Figure 41 with reference to Figures 29-30. Alternatively, construct assembly 1070 can use construct assembly 800 in the manner discussed above for use in femoral fixation at the entrance 466 and exit 474 of femoral tunnel 458, as shown in Figures 31A-32.

Construct assembly 1070 can also include a tibial fixation assembly 1074 for use in fixing the tensioned graft strands 470 relative to the entrance 452 and exit 456 of tibial tunnel 450, as shown in Figure 41. In one exemplary configuration, tibial fixation assembly 1074 can include implant 14 having a transverse bore 1078 adjacent the distal end 334 with a third flexible member construct 472 coupled thereto. In the exemplary configuration illustrated, fixation member 754 can be coupled to implant 14 via transverse bore 1078 and third flexible member construct 472, as shown in Figure 41. It should be appreciated that a notch or projection could alternatively be used on implant 14 in place of bore 1078 to provide for coupling third flexible member construct 472 and associated fixation member 754 to implant 14 during the procedure. It should also be appreciated that other tibial fixation members discussed herein can be alternatively used with tibial fixation assembly 1074 in place of implant 14.

In operation, construct assembly 1070 can be used to provide femoral and tibial fixation of graft strands 470. In the exemplary configuration illustrated, construct assembly 750 can be used to draw graft strands 470 into femoral tunnel 458 and fix graft strands 470 relative to outer surface 554 and entrance 466, as shown in Figure 41 and discussed above. The tibial fixation assembly 1074 can then be used to provide tibial fixation of the tensioned graft strands 470 relative to the entrance 452 and exit 456 of tibial tunnel 450.

In this regard, fixation member 754 can be positioned relative to the joint space 460 such that third flexible member construct assembly 472 extends into joint space 460 and down through tibial tunnel 450 to implant 14 adjacent exit 456. Once implant 14 is implanted into tibial tunnel 450 to fix the tensioned graft strands 470 relative thereto, free ends 482, 486 of third construct 472 can be tensioned to draw fixation member 754 into tibial tunnel 450 via joint space 460 and fix the tensioned graft strands 470 relative to entrance 452 of tibial tunnel 450. In the exemplary configuration illustrated, fixation member 754 can be drawn into tibial tunnel 450 such that distal end 784 is adjacent entrance 452.

While one or more specific examples have been described and illustrated, it will be understood by those skilled in the art that various changes may be made and equivalence may be substituted for elements thereof without departing from the scope of the present teachings as defined in the claims. Furthermore, the mixing and matching of features, elements and/or functions between various examples may be expressly contemplated herein so that one skilled in the art would appreciate from the present teachings that features, elements and/or functions of one example may be incorporated into another example as appropriate, unless described otherwise above. Moreover, many modifications may be made to adapt a particular situation or material to the present teachings without departing from the essential scope thereof.

## Claims

1. A tensioner system (10) for use in graft strand tensioning and fixation in a knee reconstruction procedure, comprising:
a handle assembly, the handle assembly includes a hollow outer handle body (18) and
an inner handle body (22) slidably received within the outer handle body;
a tensioner assembly (26) coupled relative to the handle assembly, the tensioner assembly including:
an arm support member (110) rotatably coupled to the handle assembly; and
first and second arm members (114) each rotatably coupled to lateral sides of the arm support member, each arm member including at least one graft attachment area (166, 174, 178) adapted to be coupled to a graft strand, the arm support member configured to rotate about an axis (132) perpendicular to a longitudinal axis (134) of the tensioner system and perpendicular to an axis (160) of rotation of each of the first and second arm members;
a drive shaft (34) slidably received through and supported by the handle assembly such that proximal and distal ends (298, 302) of the drive shaft extend beyond respective proximal and distal ends (42, 46) of the handle assembly; and
a ratcheting member (30) slidably received in the outer handle body and including a plurality of ratchet teeth (258) in selective engagement with a pawl (266) carried by the outer handle body, the ratchet teeth configured to maintain a position of the outer handle body relative to the ratcheting member upon movement of the outer handle body relative to the ratcheting member.

2. The tensioner system of claim 1, further comprising an implant (14) coupled to the distal end of the drive shaft.

3. The tensioner system of claim 1, wherein the arm support member is rotatably coupled to a distal end (82) of the inner handle body.

4. The tensioner system of claim 3, further comprising a spring (400) positioned within the outer handle body about the inner handle body and biasing the arm support member and arms in a direction toward and against the outer handle body, wherein movement of the outer handle body relative to the inner handle body increases a longitudinal length of the tensioner system.

5. The tensioner system of claim 3, wherein when the arm support member and arms are biased against the outer handle body, the arm support member and arms are locked in a non-deployed or deployed position; and
wherein when tension is applied to the tensioner assembly such that the outer handle body moves apart from the arms and arm support member, the arms and arm support member are free to independently rotate about each of their respective axes.

6. The tensioner system of claim 1, further comprising a tension release member (70) coupled to the outer handle body and in engagement with the pawl, the tension release member configured to allow engagement of the pawl with the ratchet teeth in a first position and to disengage the pawl from the ratchet teeth upon movement of the tension release member from the first position to a second position.

7. The tensioner system of claim 1, wherein the arm support member is rotatably coupled to the inner handle body at a longitudinally central portion of the arm support member such that each arm is positioned on an opposite side of and laterally spaced apart from the inner handle body and the ratcheting member.

8. The tensioner system of claim 1, wherein the ratcheting member includes a throughbore (244) slidably receiving a portion of the drive shaft, and a C-shaped distal end (218, 228) adapted to be aligned with a tibial bone tunnel and receive the drive shaft therethrough.

9. The tensioner system of claim 8, wherein the ratcheting member further comprises a bone engaging member (236) protruding from the distal end thereof beyond a bone engaging surface of the C-shaped distal end.

10. The tensioner system of claim 1, wherein the inner handle body includes a U-shaped channel (98) having an open end, and wherein the ratcheting member includes a second side opposite a first side having the ratchet teeth, the second side slidably abutting the open end of the U-shaped channel of the inner handle body.

11. The tensioner system of claim 10, wherein the drive shaft is received within the U-shaped channel of the inner handle body so as to have at least a portion surrounded by the inner handle body and the ratcheting member.

12. The tensioner system of claim 1, wherein each arm member includes a pair of graft strand attachment portions (174, 178) at opposed ends thereof such that the tensioner assembly is adapted to be coupled to four graft strands with each arm adapted to be coupled to a pair of the four graft strands.

13. The tensioner system of claim 12, wherein the attachment portions include slots (186) adapted to receive a respective suture (170) coupled to each graft strand.

14. The tensioner system of claim 2, wherein the implant includes a body (326) having a proximal end (330), a distal end (334) and an inner bore (310) extending from the proximal end partially toward the distal end, and wherein the distal end of the drive shaft is received in the bore of the implant and the proximal end of the drive shaft includes an impact plate (300).

15. The tensioner system of claim 14, wherein the implant includes a plurality of segmented portions (340) having an increasing diameter from the distal end to the proximal end.

16. The tensioner system of claim 14, wherein the implant further comprises four longitudinally extending ribs each equally circumferentially spaced apart from each other around an outer surface of the implant.

17. The tensioner system of claim 15, wherein the implant includes an angled surface (628) at the proximal end adapted to correspond to an angle of a tibial bone tunnel relative to a longitudinal axis of the tibia such that a length of the implant along a superior surface is less than a length of the implant along an opposite inferior surface.

18. The tensioner system of claim 17, further comprising a projection (732) extending radially outward from the proximal end at the superior surface of the implant.

19. The tensioner system of claim 1, wherein the inner handle body includes a scale (570) positioned about a proximal end thereof having indicia (574) indicative of an amount of tension being applied by the tensioner system.

20. The tensioner system of claim 2, wherein the handle assembly, arm support member, drive shaft and implant are cannulated.

21. The tensioner system of claim 2, further comprising:
a self-locking adjustable flexible member construct (472) having a pair of adjustable loops (514, 514') adapted to be coupled to graft strands; and
a fixation member (550) coupled to the adjustable flexible member construct;
wherein the fixation member is adapted to fix the adjustable flexible member construct relative to a bone tunnel.

## Patentansprüche

1. Spannersystem (10) zur Verwendung beim Spannen und Fixieren eines Transplantatstrangs in einem Knierekonstruktionsverfahren, umfassend:
eine Griffeinheit, wobei die Griffeinheit einen hohlen äußeren Griffkörper (18) und einen inneren Griffkörper (22), der verschiebbar in dem äußeren Griffkörper aufgenommen ist, enthält; eine Spannereinheit (26), die in Bezug auf die Griffeinheit gekoppelt ist, wobei die Spannereinheit enthält:
ein Armträgerelement (110), das drehbar mit der Griffeinheit gekoppelt ist; und ein erstes und zweites Armelement (114), die jeweils drehbar mit seitlichen Seiten des Armträgerelements gekoppelt sind, wobei jedes Armelement mindestens einen Transplantatbefestigungsbereich (166, 174, 178) enthält, der angepasst ist, um mit einem Transplantatstrang gekoppelt zu werden, das Armträgerelement konfiguriert ist, um um eine Achse (132) senkrecht zu einer Längsachse (134) des Spannersystems und senkrecht zu einer Achse (160) der Drehung von jedem des ersten und zweiten Armelements zu drehen;
eine Triebwelle (34), die verschiebbar durch die Griffeinheit aufgenommen ist und von dieser gestützt wird, sodass ein proximales und distales Ende (298, 302) der Triebwelle sich über ein jeweiliges proximales und distales Ende (42, 46) der Griffeinheit hinaus erstreckt; und
ein Rastelement (30), das verschiebbar in dem äußeren Griffkörper aufgenommen ist und eine Vielzahl von Rastzähnen (258) in selektivem Eingriff mit einer Klinke (266) enthält, die von dem äußeren Griffkörper getragen wird, wobei die Rastzähne konfiguriert sind, um eine Position des äußeren Griffkörpers in Bezug auf das Rastelement bei einer Bewegung des äußeren Griffkörpers in Bezug auf das Rastelement beizubehalten.

2. Spannersystem nach Anspruch 1, ferner umfassend ein Implantat (14), das mit dem distalen Ende der Triebwelle gekoppelt ist.

3. Spannersystem nach Anspruch 1, wobei das Armträgerelement drehbar mit einem distalen Ende (82) des inneren Griffkörpers gekoppelt ist.

4. Spannersystem nach Anspruch 3, ferner umfassend eine Feder (400), die in dem äußeren Griffkörper um den inneren Griffkörper positioniert ist und das Armträgerelement und die Arme in eine Richtung zu und gegen den äußeren Griffkörper vorspannt, wobei eine Bewegung des äußeren Griffkörpers in Bezug auf den inneren Griffkörper eine längs gerichtete Länge des Spannersystems vergrößert.

5. Spannersystem nach Anspruch 3, wobei, wenn das Armträgerelement und die Arme gegen den äußeren Griffkörper vorgespannt sind, das Armträgerelement und die Arme in einer nicht-ausgefahrenen oder ausgefahrenen Position verriegelt sind; und
wobei, wenn eine Spannung auf die Spannereinheit angewendet wird, sodass sich der äußere Griffkörper weg von den Armen und dem Armträgerelement bewegt, die Arme und das Armträgerelement frei sind, um unabhängig um jede ihrer jeweiligen Achsen zu drehen.

6. Spannersystem nach Anspruch 1, ferner umfassend ein Spannungsaufhebungselement (70), das mit dem äußeren Griffkörper gekoppelt und in Eingriff mit der Klinke ist, wobei das Spannungsaufhebungselement konfiguriert ist, um einen Eingriff der Klinke mit den Rastzähnen in einer ersten Position zu erlauben, und um die Klinke bei einer Bewegung des Spannungsaufhebungselements aus den Rastzähnen von einer ersten Position zu einer zweiten Position auszurasten.

7. Spannersystem nach Anspruch 1, wobei das Armträgerelement an einem längs gerichteten mittleren Abschnitt des Armträgerelements drehbar mit dem inneren Griffkörper gekoppelt ist, sodass jeder Arm auf einer gegenüberliegenden Seite von dem inneren Griffkörper und dem Rastelement positioniert und seitlich davon beabstandet ist.

8. Spannersystem nach Anspruch 1, wobei das Rastelement eine Durchgangsbohrung (244) enthält, die verschiebbar einen Abschnitt der Triebwelle aufnimmt, und ein C-förmiges distales Ende (218, 228), das angepasst ist, um mit einem Schienbeinknochentunnel ausgerichtet zu werden und die Triebwelle dort hindurch aufzunehmen.

9. Spannersystem nach Anspruch 8, wobei das Rastelement ferner ein Knocheneingriffselement (236) umfasst, das von dem distalen Ende davon über eine Knocheneingriffsfläche des C-förmigen distalen Endes hervorsteht.

10. Spannersystem nach Anspruch 1, wobei der innere Griffkörper einen U-förmigen Kanal (98) mit einem offenen Ende enthält, und wobei das Rastelement eine zweite Seite gegenüber einer ersten Seite mit den Rastzähnen enthält, wobei die zweite Seite verschiebbar an dem offenen Ende des U-förmigen Kanals des inneren Griffkörpers angrenzt.

11. Spannersystem nach Anspruch 10, wobei die Triebwelle in dem U-förmigen Kanal des inneren Griffkörpers aufgenommen ist, sodass mindestens ein Abschnitt von dem inneren Griffkörper und dem Rastelement umgeben ist.

12. Spannersystem nach Anspruch 1, wobei jedes Armelement ein Paar Transplantatstrang-Befestigungsabschnitte (174, 178) an gegenüberliegenden Enden davon enthält, sodass die Spannereinheit angepasst ist, um mit vier Transplantatsträngen gekoppelt zu werden, wobei jeder Arm angepasst ist, um mit einem Paar der vier Transplantatstränge gekoppelt zu werden.

13. Spannersystem nach Anspruch 12, wobei die Befestigungsabschnitte Schlitze (186) enthalten, die angepasst sind, um einen jeweiligen Faden (170) aufzunehmen, der mit jedem Transplantatstrang gekoppelt ist.

14. Spannersystem nach Anspruch 2, wobei das Implantat einen Körper (326) mit einem proximalen Ende (330), einem distalen Ende (334) und einer inneren Bohrung (310), die sich von dem proximalen Ende teilweise zu dem distalen Ende erstreckt, enthält, und wobei das distale Ende der Triebwelle in der Bohrung des Implantats aufgenommen ist und das proximale Ende der Triebwelle eine Prallplatte (300) enthält.

15. Spannersystem nach Anspruch 14, wobei das Implantat eine Vielzahl von segmentierten Abschnitten (340) mit einem zunehmenden Durchmesser von dem distalen Ende zu dem proximalen Ende enthält.

16. Spannersystem nach Anspruch 14, wobei das Implantat ferner vier sich längs erstreckende Rippen umfasst, die jeweils gleichförmig am Umfang voneinander um eine äußere Fläche des Implantats beabstandet sind.

17. Spannersystem nach Anspruch 15, wobei das Implantat eine abgewinkelte Fläche (628) an dem proximalen Ende enthält, die angepasst ist, um einem Winkel eines Schienbeinknochentunnels in Bezug auf eine Längsachse des Schienbeins zu entsprechen, sodass eine Länge des Implantats entlang einer oberen Fläche weniger ist als eine Länge des Implantats entlang einer gegenüberliegenden inneren Fläche.

18. Spannersystem nach Anspruch 17, ferner umfassend einen Vorsprung (732), der sich radial auswärts von dem proximalen Ende an der oberen Fläche des Implantats erstreckt.

19. Spannersystem nach Anspruch 1, wobei der innere Griffkörper eine Skala (570) enthält, die um ein proximales Ende davon positioniert ist und eine Markierung (574) hat, die eine Menge der Spannung angibt, die von dem Spannersystem angewendet wird.

20. Spannersystem nach Anspruch 2, wobei die Griffeinheit, das Armträgerelement, die Triebwelle und das Implantat kanüliert sind.

21. Spannersystem nach Anspruch 2, ferner umfassend:
ein selbstverriegelndes einstellbares flexibles Elementkonstrukt (472) mit einem Paar einstellbarer Schleifen (514, 514'), die angepasst sind, um mit Transplantatsträngen gekoppelt zu werden; und
ein Fixierungselement (550), das mit dem einstellbaren flexiblen Elementkonstrukt gekoppelt ist;
wobei das Fixierungselement angepasst ist, um das einstellbare flexible Elementkonstrukt in Bezug auf einen Knochentunnel zu fixieren.

## Revendications

1. Système tensionneur (10) destiné à être utilisé dans la fixation et le tensionnement de brins de greffon dans un procédé de reconstruction de genou, comprenant :
un ensemble poignée, ledit ensemble poignée comprenant un corps de poignée externe creux (18) et un corps de poignée interne (22) reçu de manière à pouvoir coulisser dans le corps de poignée externe ; un ensemble tensionneur (26) couplé à l'ensemble poignée, ledit ensemble tensionneur comprenant :
un élément support de bras (110) couplé de manière à pouvoir tourner à l'ensemble poignée ; et des premier et second éléments bras (114) couplés chacun de manière à pouvoir tourner à des côtés latéraux de l'élément support de bras, chaque élément bras comprenant au moins une zone de fixation de greffon (166, 174, 178) adaptée pour être couplée à un brin de greffon, ledit élément support de bras conçu pour tourner autour d'un axe (132) perpendiculaire à l'axe longitudinal (134) du système tensionneur et perpendiculaire à un axe (160) de rotation de chacun des premier et second éléments de bras ;
un arbre d'entraînement (34) reçu de manière à pouvoir coulisser à travers l'ensemble poignée et étant supporté par celui-ci de sorte que des extrémités proximales et distales (298, 302) de l'arbre d'entraînement s'étendent au-delà des extrémités proximales et distales respectives (42, 46) de l'ensemble poignée ; et un élément d'encliquetage (30) reçu de manière à pouvoir coulisser dans le corps de poignée externe et comprenant une pluralité de dents d'encliquetage (258) en prise de manière sélective avec un cliquet (266) porté par le corps de poignée externe, les dents d'encliquetage étant conçues pour maintenir une position du corps de poignée externe par rapport à l'élément d'encliquetage lors du déplacement du corps de poignée externe par rapport à l'élément d'encliquetage.

2. Système tensionneur selon la revendication 1, comprenant en outre un implant (14) couplé à l'extrémité distale de l'arbre d'entraînement.

3. Système tensionneur selon la revendication 1, ledit élément support de bras étant couplé de manière à pouvoir tourner à une extrémité distale (82) du corps de poignée interne.

4. Système tensionneur selon la revendication 3, comprenant en outre un ressort (400) positionné dans le corps de poignée externe autour du corps de poignée interne et sollicitant l'élément support de bras et les bras selon une direction vers et contre le corps de poignée externe, un déplacement du corps de poignée externe par rapport au corps de poignée interne augmentant une longueur longitudinale du système tensionneur.

5. Système tensionneur selon la revendication 3, lorsque l'élément support de bras et les bras sont sollicités contre le corps de poignée externe, ledit élément support de bras et lesdits bras étant verrouillés dans une position non-déployée ou déployée ; et
lorsqu'une tension est appliquée à l'ensemble tensionneur de sorte que le corps de poignée externe s'écarte des bras et de l'élément support de bras, lesdits bras et ledit élément support de bras étant libres de tourner indépendamment autour de leurs axes respectifs.

6. Système tensionneur selon la revendication 1, comprenant en outre un élément de libération de tension (70) couplé au corps de poignée externe et en prise avec le cliquet, ledit élément de libération de tension étant conçu pour permettre la mise en prise du cliquet avec les dents d'encliquetage dans une première position et le désengagement du cliquet des dents d'encliquetage lors d'un déplacement de l'élément de libération de tension depuis la première position jusqu'à une seconde position.

7. Système tensionneur selon la revendication 1, ledit élément support de bras étant couplé de manière à pouvoir tourner au corps de poignée interne au niveau d'une partie centrale longitudinalement de l'élément support de bras de sorte que chaque bras soit positionné sur un côté opposé du corps de poignée interne et de l'élément d'encliquetage et étant espacé latéralement de ceux-ci.

8. Système tensionneur selon la revendication 1, ledit élément d'encliquetage comprenant un alésage traversant (244) recevant de manière coulissante une partie de l'arbre d'entraînement, et une extrémité distale en forme de C (218, 228) adaptée pour s'aligner avec un tunnel osseux tibial et pour recevoir l'arbre d'entraînement à travers elle.

9. Système tensionneur selon la revendication 8, ledit élément d'encliquetage comprenant en outre un élément de mise en prise avec l'os (236) faisant saillie depuis l'extrémité distale de celui-ci au-delà d'une surface de mise en prise avec l'os de l'extrémité distale en forme de C.

10. Système tensionneur selon la revendication 1, ledit corps de poignée interne comprenant un canal en forme de U (98) comportant une extrémité ouverte et ledit élément d'encliquetage comprenant un second côté opposé à un premier côté possédant les dents d'encliquetage, ledit second côté en butée de manière coulissante contre l'extrémité ouverte du canal en forme de U du corps de poignée interne.

11. Système tensionneur selon la revendication 10, ledit arbre d'entraînement étant reçu dans le canal en forme de U du corps de poignée interne de façon à avoir au moins une partie entourée par le corps de poignée interne et l'élément d'encliquetage.

12. Système tensionneur selon la revendication 1, chaque élément bras comprenant une paire de parties de fixation de brins de greffon (174, 178) au niveau des extrémités opposées de celui-ci de sorte que l'ensemble tensionneur soit adapté pour être couplé à quatre brins de greffon avec chaque bras adapté pour être couplé à une paire de quatre brins de greffon.

13. Système tensionneur selon la revendication 12, lesdites parties de fixation comprenant des fentes (186) adaptées pour recevoir une suture respective (170) couplée à chaque brin de greffon.

14. Système tensionneur selon la revendication 2, ledit implant comprenant un corps (326) possédant une extrémité proximale (330), une extrémité distale (334) et un alésage interne (310) s'étendant à partir de l'extrémité proximale partiellement vers l'extrémité distale et ladite extrémité distale de l'arbre d'entraînement étant reçue dans l'alésage de l'implant et ladite extrémité proximale de l'arbre d'entraînement comprenant une plaque d'impact (300).

15. Système tensionneur selon la revendication 14, ledit implant comprenant une pluralité de parties segmentées (340) possédant un diamètre augmentant depuis l'extrémité distale jusqu'à l'extrémité proximale.

16. Système tensionneur selon la revendication 14, ledit implant comprenant en outre quatre nervures s'étendant longitudinalement espacées chacune circonférentiellement de manière uniforme les unes des autres autour d'une surface externe de l'implant.

17. Système tensionneur selon la revendication 15, ledit implant comprenant une surface inclinée (628) au niveau de l'extrémité proximale adaptée pour correspondre à un angle d'un tunnel osseux tibial par rapport à un axe longitudinal du tibia de sorte qu'une longueur de l'implant le long d'une surface supérieure soit inférieure à une longueur de l'implant le long d'une surface inférieure opposée.

18. Système tensionneur selon la revendication 17, comprenant en outre une saillie (732) s'étendant radialement vers l'extérieur depuis l'extrémité proximale au niveau de la surface supérieure de l'implant.

19. Système tensionneur selon la revendication 1, ledit corps de poignée interne contenant une échelle graduée (570) positionnée autour de l'extrémité proximale de celui-ci possédant des signes (574) indiquant la quantité de tension appliquée par le système tensionneur.

20. Système tensionneur de la revendication 2, ledit ensemble poignée, ledit élément support de bras, ledit arbre d'entraînement et ledit implant étant canulés.

21. Système tensionneur selon la revendication 2, comprenant en outre :
une construction d'élément souple ajustable à verrouillage automatique (472) possédant une paire de boucles ajustables (514, 514') adaptées pour être couplées aux brins de greffon ; et un élément de fixation (550) couplé à la construction d'élément souple ajustable ; ledit élément de fixation étant adapté pour fixer la construction d'élément souple ajustable par rapport à un tunnel osseux.
